(19) Europäisches Patentamt / European Patent Office / Office européen des brevets

(11) **EP 4 442 719 A1**

(12) **EUROPEAN PATENT APPLICATION**
published in accordance with Art. 153(4) EPC


(43) Date of publication:
**09.10.2024 Bulletin 2024/41**

(21) Application number: **22901412.1**

(22) Date of filing: **01.12.2022**

(51) International Patent Classification (IPC):
***C08F 220/10** (2006.01)* ***C07D 279/36** (2006.01)*
***C08F 212/32** (2006.01)* ***C08K 5/17** (2006.01)*
***C08L 31/06** (2006.01)* ***C09K 15/32** (2006.01)*

(52) Cooperative Patent Classification (CPC):
**C07D 279/36; C08F 212/32; C08F 220/10; C08K 5/17; C08L 31/06; C09K 15/32**

(86) International application number:
**PCT/JP2022/044383**

(87) International publication number:
**WO 2023/100982 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.12.2021 JP 2021195998**
**14.04.2022 JP 2022066753**



(71) Applicant: **Unimatec Co., Ltd.**
**Tokyo 105-0012 (JP)**

(72) Inventor: **SAITO Satoru**
**Kitaibaraki-shi, Ibaraki 319-1593 (JP)**

(74) Representative: **Kraus & Lederer PartGmbB**
**Thomas-Wimmer-Ring 15**
**80539 München (DE)**


(54) **ACRYLIC ELASTOMER COPOLYMER**


(57) An acrylic elastomer copolymer comprising a copolymerizable antioxidant represented by the general formula:

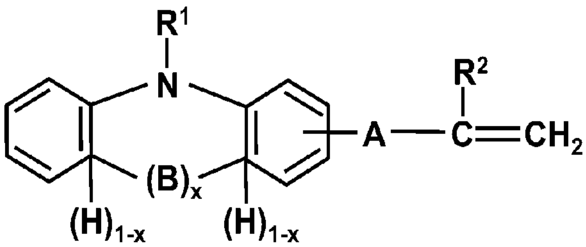

[I]

($R^1$: a $C_{1-20}$ aliphatic hydrocarbon group, a $C_{7-20}$ aralkyl group, or a $C_{2-20}$ acyl group, $R^2$: a hydrogen atom or a methyl group, A: a direct bond or a divalent organic group, B: a direct bond, an oxygen atom, a sulfur atom, a sulfoxide group, or a sulfone group, and x: 0 or 1), an alkyl (meth)acrylate monomer and/or an alkoxyalkyl (meth)acrylate monomer, and a crosslinking site monomer. The acrylic elastomer copolymer is stabilized against thermal oxidative deterioration.


EP 4 442 719 A1

**Description**

TECHNICAL FIELD

The present invention relates to an acrylic elastomer copolymer. More particularly, the present invention relates to an acrylic elastomer copolymer that is stabilized against thermal oxidative deterioration.

BACKGROUND ART

Emission regulations for carbon dioxide, NOx gas, and the like emitted from internal combustion engines, such as automobile engines typified by gasoline engines and diesel engines, are tending to become increasingly strict. As a countermeasure thereof, automobile engines are required to have higher output, higher thermal efficiency, and lower or harmless emissions. The temperature in the engine compartment is tended to increase to increase. Along with this trend, polymer materials, such as rubber and plastic, used in the surrounding area are required to have further improved heat resistance.
As specific examples, vehicles equipped with a turbocharger system for the purpose of improving the fuel efficiency of the engine are becoming widespread. Since air guided from the turbocharger to the intercooler and engine has high temperature and high pressure, high heat resistance is required for rubber hose materials that transport the air.
As described above, with the demand for higher operating temperatures and longer life of polymer materials used in automobile engines, it is common practice to add appropriate antioxidants (oxidation inhibitors) to rubber members or plastic members to improve their heat resistance.
Phenol-based antioxidants and amine-based antioxidants are used as antioxidants for rubber members, and amine-based antioxidants are particularly used for rubber members that are used in higher temperature environments.
For example, in the case of acrylic rubber, amine-based antioxidants typified by 4,4'-bis($\alpha$,$\alpha$-dimethylbenzyl)diphenylamine are used as antioxidants (Patent Documents 1 to 6).
However, even with the amine-based antioxidants mentioned above, it is not possible to fully satisfy the recent issues, such as heat resistance requirements.
In recent years, phenothiazine-based antioxidants are said to be effective as antioxidants for rubber materials. As a rubber material that has excellent vulcanization characteristics, mechanical characteristics, and heat aging characteristics, and that is particularly suitable for use in anti-vibration rubber, Patent Document 7 discloses one comprising (A) a diene-based rubber, (B) a bismaleimide compound, and (C) the following phenothiazine compound:

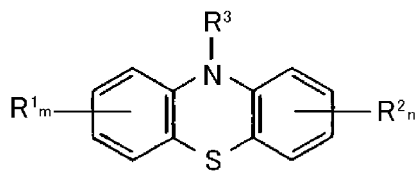

$R^1$, $R^2$: a hydrogen atom, a $C_1$-$C_8$ alkyl group that may be substituted with an aromatic ring, an alkoxy group, a halogen atom, or a cyano group
$R^3$: a hydrogen atom, a $C_1$-$C_6$ chain or cyclic alkyl group, a vinyl group, or an aromatic group
m, n: 0 to 2

A phenothiazine compound in which the sulfur atom at position 5 is $-SO_2-$ is also known and described, for example, in Patent Document 8.
Patent Document 8 discloses a condensed heterocyclic compound represented by the following general formula and an organic material composition comprising the same, and states that it is possible to impart high processing stability, heat resistance, and long life to organic materials such as polymer that is susceptible to oxidative, thermal, or photo-induced breakdown.

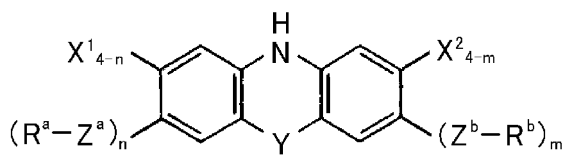

Y: a chemical single bond, -S(=O)-, or $-SO_2-$

$R^a$, $R^b$: a $C_1$-$C_{30}$ organic group that may have a substituent
$Z^a$, $Z^b$: a chemical single bond or $-SO_2-$
$X^1$, $X^2$: a hydrogen atom, a halogen atom, an alkyl group, a cyano group, a nitro group, $-OR^1$, $-O-CO-R^1$, $-CO-OR^1$, $-O-CO-OR^1$, $-NR^2R^3$, $-NR^2-CO-R^1$, $-CO-NR^2R^3$, or $-O-CO-NR^2R^3$
n, m: 0 to 2, provided that one of them is not 0.

In addition, attempts have been made to increase the molecular weight and melting point of amine-based antioxidants; however, there are problems, such as poor dispersibility in rubber and poor migration within the rubber.
Furthermore, antioxidants with polymerizable unsaturated groups have been put on the market for the purpose of preventing the volatilization of antioxidants and extending the life of rubber members in high temperature environments.
Examples of such antioxidants include Nocrac G-1 (produced by Ouchi Shinko Chemical Industrial Co., Ltd.) and APMA (produced by Seiko Chemical Co., Ltd.) (Non-Patent Documents 1 and 2).

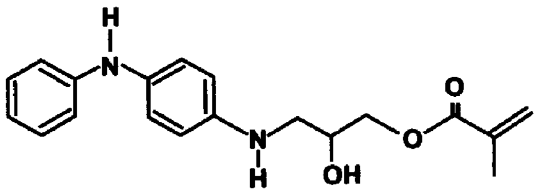

Nocrac G-1

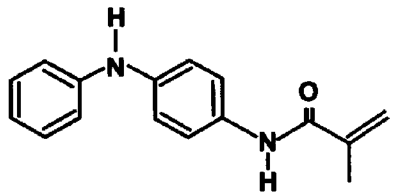

APMA

However, with the above antioxidants, radical copolymerization with a polymerizable unsaturated monomer is practically difficult due to the radical polymerization inhibitory effect of the diphenylamino group (Patent Document 9).
Furthermore, several methods are disclosed for introducing a diphenylamino structure into a polymer by the modification reaction of elastomeric polymer. For example, the following methods are known: a method in which a diphenylamino group is introduced after hydroformylation of the side chain of an elastomer having an olefine-based unsaturated group (Patent Document 10), and a method in which a diphenylamino group is introduced after maleic anhydride is added to a diene-based copolymer in the presence of a free radical generator (Patent Document 11). However, these methods further require a modification step of introducing a diphenylamino group after producing a base copolymer, which is not practical in terms of production cost.
A technique has also been disclosed in which an antioxidant component is chemically bonded to the reaction site of the main chain of an elastomeric copolymer by using 4-aminodiphenylamine in combination during crosslinking (Patent Document 12); however, there is a concern that it may deteriorate compression set resistance characteristics, and its use is limited.

PRIOR ART DOCUMENT

PATENT DOCUMENT

Patent Document 1 : JP-A-11-21411
Patent Document 2 : WO 2007/005458 A1
Patent Document 3 : JP-A-2010-254579
Patent Document 4 :WO 2006/001299 A1
Patent Document 5 : JP-A-2011-032390
Patent Document 6 :WO 2011/58918 A1
Patent Document 7 : JP-A-2015-227402
Patent Document 8 :WO 2011/093443 A1
Patent Document 9 : JP-A-2009-209268
Patent Document 10 : JP-A-4-264106
Patent Document 11 : JP-A-5-230132
Patent Document 12 :WO 2020/158132 A1

NON-PATENT DOCUMENTS

Non-Patent Document 1 : Rubber Chem.Technol., Vol. 46, pp. 106 (1973)
Non-Patent Document 2 : Rubber Chem.Technol., Vol. 52, pp. 883 (1979)

OUTLINE OF THE INVENTION

PROBLEM TO BE SOLVED BY THE INVENTION

The present invention was made in view of the problems described above, and an object thereof is to provide an acrylic elastomer copolymer that is stabilized against thermal oxidative deterioration.

MEANS FOR SOLVING THE PROBLEM

The present invention is an acrylic elastomer copolymer comprising a copolymerizable antioxidant represented by the general formula:

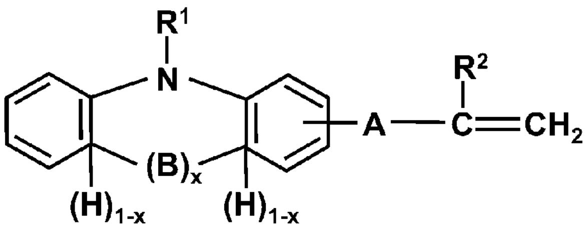

〔I〕

(wherein $R^1$ is a $C_{1-20}$ aliphatic hydrocarbon group, a $C_{7-20}$ aralkyl group, or a $C_{2-20}$ acyl group, $R^2$ is a hydrogen atom or a methyl group, A is a direct bond or a divalent organic group, B is a direct bond, an oxygen atom, a sulfur atom, a sulfoxide group, or a sulfone group, and x is 0 or 1), an alkyl (meth)acrylate monomer and/or an alkoxyalkyl (meth)acrylate monomer, and a crosslinking site monomer.

EFFECT OF THE INVENTION

Since the acrylic elastomer copolymer of the present invention contains an antioxidant component in the polymer side chain, and the copolymer itself is stabilized against thermal oxidative deterioration, it is not necessary to newly add a phenol-based antioxidant or an amine-based antioxidant in the production and processing steps. Therefore, it is possible to save the steps of measuring, adding, and mixing such additives in the production and processing steps, and it is also possible to eliminate concerns about defects caused by poor dispersion of these additives.

Since the acrylic elastomer copolymer of the present invention contains an antioxidant component in the polymer chain, as described above, the process of inactivating peroxy radicals generated by thermal oxidative deterioration can be considered as an intramolecular reaction. In contrast, when a generally used phenol-based antioxidant or amine-based antioxidant is added from the outside, the process is an intermolecular reaction. As a result, if the same level of anti-heat aging properties is desired, the amount of the antioxidant component (copolymerizable antioxidant) used in the present invention can be reduced to 50% or less of the amount of a conventionally used phenol-based antioxidant or amine-based antioxidant.

Moreover, for example, when the acrylic elastomer copolymer of the present invention is produced by emulsion polymerization, the polymer emulsion can be prevented from deteriorating during storage due to its own oxidative deterioration prevention effect.

Furthermore, in the hot air drying step or extrusion drying step after the copolymer coagulation step, the acrylic elastomer copolymer can be prevented from deteriorating due to its own thermal (oxidative) deterioration prevention effect, and also allows for the subsequent long-term storage in the air. That is, the acrylic elastomer copolymer is stabilized against thermal deterioration or thermal oxidative deterioration caused by thermal history in the production process and storage stage, and is therefore effective in improving productivity and storage stability.

Furthermore, when using an acrylic elastomer molded member obtained by crosslinking the acrylic elastomer copolymer of the present invention, the antioxidant component is chemically bonded to the rubber molecules; thus, the antioxidant component can be prevented from being volatilized into the air or being extracted by liquid media such as

oils, fats, and organic solvents. As a result, it is possible to extend the life of the acrylic elastomer molded member under various deterioration environments.

The elastomer copolymer of the present invention in which a copolymerizable antioxidant is copolymerized can be mixed with an elastomer copolymer in which no antioxidant is compounded or an antioxidant component is not chemically bonded to the polymer chain, that is, an elastomer copolymer that is not stabilized against thermal oxidative deterioration, thereby imparting anti-aging properties to its crosslinked product. Therefore, the elastomer copolymer of the present invention is expected to function as a polymer antioxidant.

BRIEF DESCRIPTION OF DRAWINGS

Fig. 1: A schematic diagram of the rate of change in strength at break change over time of acrylic rubber crosslinked product at 190°C (Example 5: -●-, Example 6: -♦-, Comparative Example 1: --●--, Comparative Example 2: --+--; Figs 1 to 4 common).

Fig. 2: A schematic diagram of the rate of change in elongation at break change over time of acrylic rubber crosslinked product at 190°C.

Fig. 3: A schematic diagram of the rate of change in strength at break change over time of acrylic rubber crosslinked product at 175°C.

Fig. 4: A schematic diagram of the rate of change in elongation at break change over time of acrylic rubber crosslinked product at 175°C.

Fig. 5: A comparison of the rate of change in strength at break after an air heating aging test (190°C, 200 hours) in the air (white bar) and the rate of change in strength at break when the same air heating aging test (190°C, 200 hours) was performed after dipping in IRM 903 oil (black bar) in Examples 5 to 8 and Comparative Examples 1 and 2.

Fig. 6: A comparison of the rate of change in elongation at break after an air heating aging test (190°C, 200 hours) in the air (white bar) and the rate of change in elongation at break when the same air heating aging test (190°C, 200 hours) was performed after dipping in IRM 903 oil (black bar) in Examples 5 to 8 and Comparative Examples 1 and 2.

Fig. 7: A schematic diagram of the rate of change in strength at break change over time of acrylic rubber crosslinked product at 190°C (Example 10: -●-, Comparative Example 3: --▲--, Comparative Example 4: --+--; Figs 7 to 8 common).

Fig. 8: A schematic diagram of the rate of change in elongation at break change over time of acrylic rubber crosslinked product at 190°C.

Fig. 9: A comparison of the rate of change in strength at break after an air heating aging test (190°C, 200 hours) in the air (white bar) and the rate of change in strength at break when the same air heating aging test (190°C, 200 hours) was performed after dipping in IRM 903 oil (black bar) in Example 10 and Comparative Examples 3 and 4.

Fig. 10: A comparison of the rate of change in elongation at break after an air heating aging test (190°C, 200 hours) in the air (white bar) and the rate of change in elongation at break when the same air heating aging test (190°C, 200 hours) was performed after dipping in IRM 903 oil (black bar) in Example 10 and Comparative Examples 3 and 4.

Fig. 11: A comparison of the rate of change in strength at break after an air heating aging test (190°C, 300 hours) in the air (white bar) and the rate of change in strength at break when the combined test comprising air heating (175°C, 150 hours), dipping in IRM 903 and air heating (190°C, 300 hours) (black bar) was performed in Example 10 and Comparative Examples 3 and 4.

Fig. 12: A comparison of the rate of change in elongation at break after an air heating aging test (190°C, 300 hours) in the air (white bar) and the rate of change in elongation at break when the combined test comprising air heating (175°C, 150 hours), dipping in IRM 903 and air heating (190°C, 300 hours) (black bar) was performed in Example 10 and Comparative Examples 3 and 4.

Fig. 13: A schematic diagram of the rate of change in strength at break change over time of acrylic rubber crosslinked product at 190°C (Example 12: -●-, Comparative Example 5: --●--, Comparative Example 6: --▲--, Comparative Example 7: --1--; Figs 13 to 14 common).

Fig. 14: A schematic diagram of the rate of change in elongation at break change over time of acrylic rubber crosslinked product at 190°C.

Fig. 15: A schematic diagram of the rate of change in strength at break change over time of acrylic rubber crosslinked product at 175°C (Example 14: -●-, Comparative Example 8: --▲--, Comparative Example 9: --♦--; Figs 15 to 16 common).

Fig. 16: A schematic diagram of the rate of change in elongation at break change over time of acrylic rubber crosslinked product at 175°C.

Fig. 17: A schematic diagram of the rate of change in strength at break change over time of acrylic rubber crosslinked

product at 175°C (Example 15: -●-, Comparative Example 10: --▲--, Comparative Example 11: --+--; Figs 17 to 18 common).

Fig. 18: A schematic diagram of the rate of change in elongation at break change over time of acrylic rubber crosslinked product at 175°C.

Fig. 19: A comparison of the rate of change in elongation at break after an air heating aging test (175°C, 300 hours) (white bar) and the rate of change in elongation at break after the combined test comprising dipping in IRM 903 (150°C, 168 hours)-air heating (175°C, 300 hours) (black bar) in Examples 18 and 19 and Comparative Example 12.

Fig. 20: A comparison of the rate of change in strength at break after an air heating aging test (190°C, 200 hours) (white bar) and the rate of change in strength at break after the combined test comprising dipping in IRM 903 (150°C, 168 hours)-air heating (190°C, 200 hours) (black bar) in Examples 25 to 27 and Comparative Examples 13 to 15.

Fig. 21: A comparison of the rate of change in elongation at break after an air heating aging test (190°C, 200 hours) (white bar) and the rate of change in elongation at break after the combined test comprising dipping in IRM 903 (150°C, 168 hours)-air heating (190°C, 200 hours) (black bar) in Examples 25 to 27 and Comparative Examples 13 to 15.

EMBODIMENTS FOR CARRYING OUT THE INVENTION

The copolymerizable antioxidant used in the present invention is a compound synthesized by using diphenylamine, 4-aminodiphenylamine, phenothiazine or 5,5-dioxide thereof, 2-acetylphenothiazine, or the like as a starting material, having a diphenylamine structure as a basic skeleton in which the hydrogen atom on the amino group is replaced by an alkyl group, an aralkyl group, or an acyl group, and having a polymerizable unsaturated group, and is represented by the general formula [I]. Here, (meth)acrylate refers to acrylate or methacrylate.

Specifically, an acrylic elastomer copolymer represented by the following general formula [I] is used.

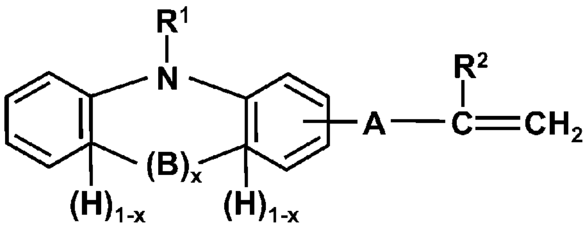

[I]

wherein $R^1$ is a $C_{1-20}$ aliphatic hydrocarbon group, a $C_{7-20}$ aralkyl group, or a $C_{2-20}$ acyl group, $R^2$ is a hydrogen atom or a methyl group, A is a direct bond or a divalent organic group, B is a direct bond, an oxygen atom, a sulfur atom, a sulfoxide group, or a sulfone group, and x is 0 or 1.

Specific examples of the $C_{1-20}$ aliphatic hydrocarbon group include primary hydrocarbon groups, such as a methyl group, an ethyl group, an n-propyl group, an n-butyl group, an n-pentyl group, an n-hexyl group, an n-heptyl group, an n-octyl group, a 2-ethylhexyl group, an n-nonyl group, an n-undecyl group, an n-pentadecyl group, an n-heptadecyl group, and an n-octadecyl group;

secondary hydrocarbon groups, such as an isopropyl group, a 2-butyl group, a 2-pentyl group, a 3-pentyl group, a 2-hexyl group, a 3-hexyl group, a 2-heptyl group, a 3-heptyl group, a 4-heptyl group, a 2-octyl group, a 3-octyl group, and a 4-octyl group;

tertiary hydrocarbon groups, such as a tertiary butyl group, a 1,1-dimethyl-1-propyl group, a 1,1-dimethyl-1-butyl group, a 1,1-dimethyl-1-pentyl group, a 1,1-dimethyl-1-hexyl group, a 3-methyl-3-pentyl group, a 3-ethyl-3-pentyl group, and a 3-methyl-3-hexyl group;

alicyclic hydrocarbon groups, such as a cyclopropyl group, a cyclobutyl group, a cyclopentyl group, a cyclohexyl group, a cycloheptyl group, a 1-methyl-1-cyclopentyl group, and a 1-methyl-1-cyclohexyl group;

a 1-adamantyl group, and the like.

Examples of the $C_{7-20}$ aralkyl group include a benzyl group, a 1-phenylethyl group, and the like.

The $C_{2-20}$ acyl group is represented by the following general formula:

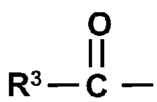

wherein $R^3$ is a $C_{1-19}$ aliphatic hydrocarbon group or a $C_{6-19}$ aromatic hydrocarbon group, and the $C_{1-19}$ aliphatic hydrocarbon group is synonymous with $R^1$ excluding $C_{20}$ aliphatic hydrocarbon groups. Examples of the $C_{6-19}$ aromatic hydrocarbon group include a phenyl group, a naphthyl group, an anthracenyl group, and the like.

A is a direct bond or a divalent organic group, and the divalent organic group is not particularly limited as long as it does not inhibit the polymerization reaction. A is preferably a direct bond, a group $-(CH_2)_nO(C{=}O)-$ (n: 1 to 5), or a group - $NH(C{=}O)(CH_2)_nO(C{=}O)-$(n: 1 to 5).

When x is 0, the copolymerizable antioxidant is represented by the following general formula [II]:

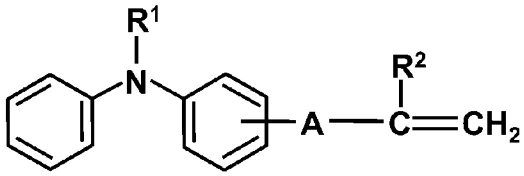

[II]

Specific examples include the following:

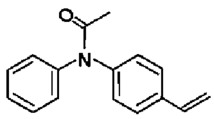

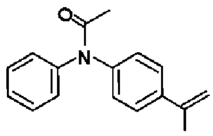

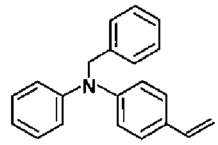

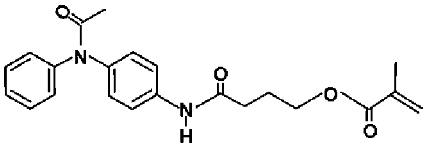

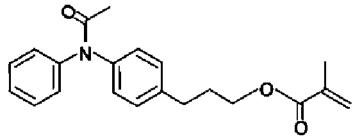

When x is 1, the copolymerizable antioxidant is represented by the following general formula [III]:

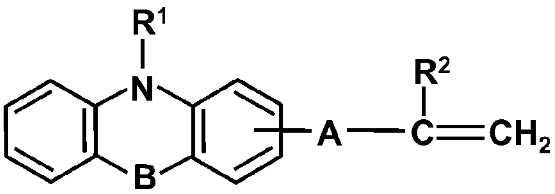

[III]

Specific examples include the following:

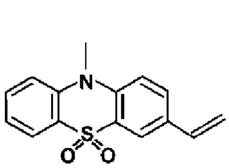

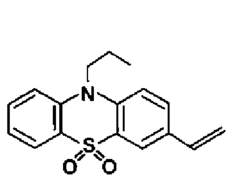

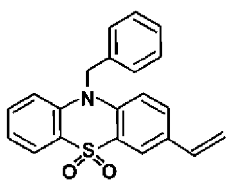

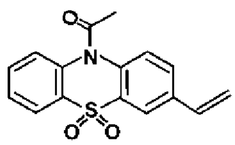

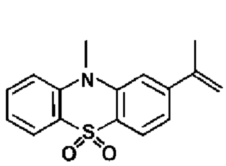

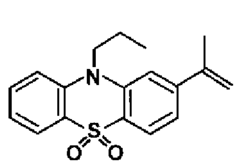

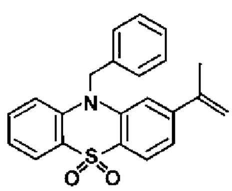

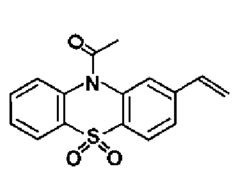

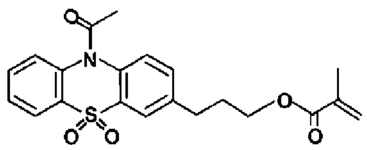
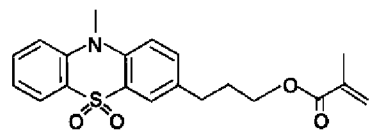

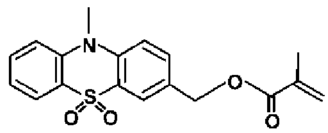
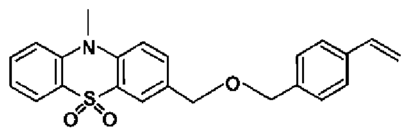

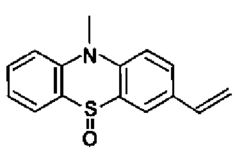
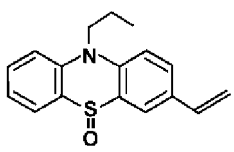
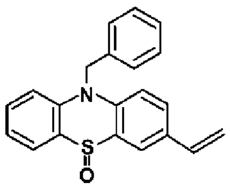
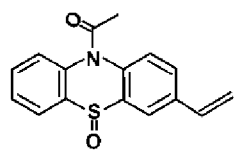

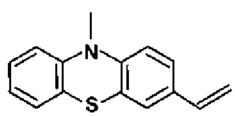
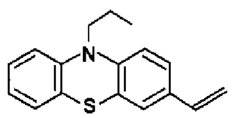
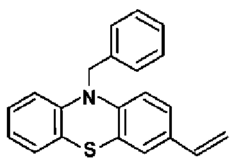
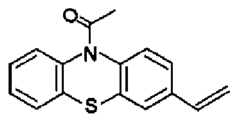

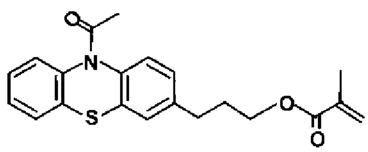
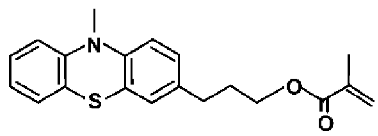

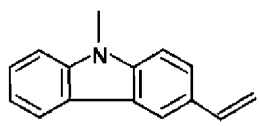
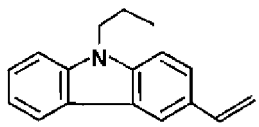
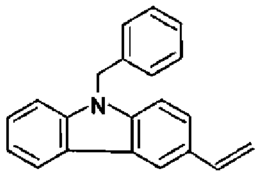
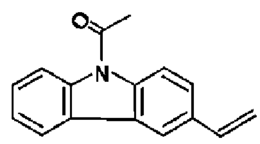

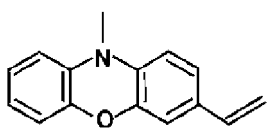
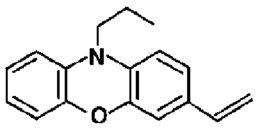
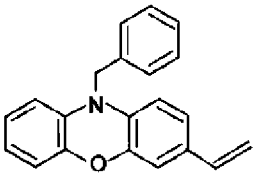
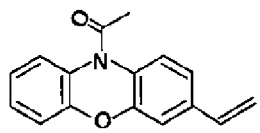

In the copolymerization of the copolymerizable antioxidant and the polymerizable unsaturated monomer, the copolymerizable antioxidant [I] is used in an amount of about 0.005 to 5 parts by weight, preferably about 0.01 to 3 parts by weight, in 100 parts by weight of the monomer mixture. The mole fraction of the copolymerizable antioxidant in the copolymer is about 0.002 to 2 mol%, preferably about 0.004 to 1 mol%. If the copolymerizable antioxidant is used at a ratio less than this range, a sufficient anti-aging effect cannot be expected. In contrast, even if the copolymerizable antioxidant is used at a ratio greater than this range, no improvement in the anti-aging effect is expected, and it is uneconomical.

As the alkyl (meth)acrylate monomer and/or alkoxyalkyl (meth)acrylate monomer that constitutes the acrylic elastomer copolymer of the present invention, at least one (meth)acrylate selected from alkyl (meth)acrylate containing a $C_{1-20}$ alkyl group, aralkyl (meth)acrylate containing a $C_{7-20}$ aralkyl group, and alkoxyalkyl (meth)acrylate containing a $C_{2-20}$ alkoxyalkyl group is used.

Examples of alkyl (meth)acrylate include methyl (meth)acrylate, ethyl (meth)acrylate, propyl (meth)acrylate, isopropyl (meth)acrylate, n-butyl (meth)acrylate, n-hexyl (meth)acrylate, 2-ethylhexyl (meth)acrylate, n-octyl (meth)acrylate, cyclohexyl (meth)acrylate, n-decyl (meth)acrylate, n-dodecyl (meth)acrylate, n-octadecyl (meth)acrylate, and the like are used.

Examples of aralkyl (meth)acrylate include benzyl (meth)acrylate and the like.

Moreover, examples of alkoxyalkyl (meth)acrylate include methoxymethyl (meth)acrylate, methoxyethyl (meth)acrylate, ethoxyethyl (meth)acrylate, n-butoxyethyl (meth)acrylate, ethoxypropyl (meth)acrylate, methoxyethoxyethyl (meth)acrylate, ethoxyethoxyethyl (meth)acrylate, polyethylene glycol monomethyl ether (meth)acrylate, polypropylene glycol monoethyl ether (meth)acrylate, and the like.

As the crosslinking site monomer that constitutes the acrylic elastomer copolymer of the present invention, an α,β-unsaturated carboxylic acid monomer, an active chlorine-containing unsaturated monomer, or an epoxy group-containing unsaturated monomer is used.

Examples of the α,β-unsaturated carboxylic acid monomer include monobasic α,β-unsaturated carboxylic acids, dibasic α,β-unsaturated carboxylic acids, or dibasic α,β-unsaturated carboxylic acid monoalkyl esters.

Examples of monobasic α,β-unsaturated carboxylic acid include acrylic acid , methacrylic acid and the like.

Examples of dibasic α,β-unsaturated carboxylic acid include maleic acid, fumaric acid, itaconic acid, citraconic acid and the like.

Examples of dibasic α,β-unsaturated carboxylic acid monoalkyl ester include monoalkyl esters of maleic acid, fumaric acid, itaconic acid, and citraconic acid. Specific examples include monomethyl maleate, monoethyl maleate, mono n-propyl maleate, monoisopropyl maleate, mono n-butyl maleate, monoisobutyl maleate, mono n-hexyl maleate, monocyclohexyl maleate, monomethyl fumarate, monoethyl fumarate, mono n-propyl fumarate, monoisopropyl fumarate, mono n-butyl fumarate, monoisobutyl fumarate, mono n-hexyl fumarate, monocyclohexyl fumarate, and the like.

Examples of active chlorine-containing unsaturated monomer include 4-chloromethyl styrene, vinyl chloroacetate, and the like.

Examples of epoxy group-containing unsaturated monomer include glycidyl acrylate, glycidyl methacrylate, and the like.

In the acrylic elastomer copolymer of the present invention, the crosslinking site monomer is copolymerized at a ratio of 0.1 to 5 wt%, preferably 0.5 to 3 wt%.

Moreover, in addition to these main components of the acrylic elastomer copolymer of the present invention, other polymerizable unsaturated monomers can be used, if necessary.

Examples of polymerizable unsaturated monomer include styrene, α-methylstyrene, 3-methylstyrene, 4-methylstyrene, 1-vinylnaphthalene, 2-vinylnaphthalene, acrylonitrile, methacrylonitrile, acrylic acid amide, vinyl acetate, methyl vinyl ether, ethyl vinyl ether, ethylene, propylene, piperylene, butadiene, isoprene, chloroprene, cyclopentadiene, vinyl chloride, vinylidene chloride, and the like.

The acrylic elastomer copolymer is produced by a general method for copolymerizing acrylic rubber. The copolymerization reaction can be carried out by any method, such as an emulsion polymerization method, a suspension polymerization method, a solution polymerization method, or a bulk polymerization method.

For example, when an emulsion polymerization method or a suspension polymerization method is used, and the reaction is carried out at a reaction temperature of about -10 to 100°C, preferably about 5 to 80°C.

Examples of the polymerization initiator for the reaction include organic peroxides or organic hydroperoxides, such as benzoyl peroxide, dicumyl peroxide, tert-butyl hydroperoxide, cumyl hydroperoxide and p-methylene hydroperoxide; diazo compounds, such as azobisisobutyronitrile and azobisisobutylamidine; ammonium salts represented by ammonium persulfate; peroxide salts, such as sodium salts and potassium salts; and the like. These are used singly or as a redox system.

As an emulsifier used in the particularly preferable emulsion polymerization method, an anionic or nonionic surfactant is used as an aqueous solution or the like whose pH is optionally adjusted by acid or base, and which is formed into a buffer solution by using an inorganic salt.

The polymerization reaction is continued until the conversion rate of the monomer mixture reaches 90% or more. The obtained aqueous latex is coagulated by a salt-acid coagulation method, a method using a salt, such as calcium chloride, magnesium sulfate, sodium sulfate, or ammonium sulfate, a method using a boron compound, such as boric acid or borax, a coagulation method by heat, a freeze coagulation method, or the like. The obtained copolymer is sufficiently washed with water and dried. This acrylic rubber has Mooney viscosity $ML_{1+4}$ (100°C) of about 5 to 100, preferably about 20 to 80.

The copolymerizable antioxidant [I] can be copolymerized with a polymerizable unsaturated monomer other than alkyl (meth)acrylate monomers and/or alkoxyalkyl (meth)acrylate monomers, and can be applied to the production of elastomeric copolymers, such as styrene-butadiene rubber (SBR), chloroprene rubber (CR), ethylene-vinyl acetate rubber (EVA), nitrile rubber (NBR), hydrogenated nitrile rubber (H-NBR), and ethylene-methyl acrylate rubber (AEM). The polymerization reaction is preferably radical polymerization or anionic polymerization.

The elastomer copolymer of the present invention is crosslinked by a crosslinking agent according to the type of crosslinking site monomer used, and a crosslinkable acrylic elastomer copolymer can be formed.

For example, when the crosslinking site monomer is an $\alpha$,$\beta$-unsaturated carboxylic acid monomer, a polyvalent amine compound is used as the crosslinking agent.

Examples of the polyvalent amine crosslinking agent include hexamethylenediamine, hexamethylenediamine carbamate, N,N'-dicinnamylidene-1,6-hexanediamine, 4,4'-bis(aminocyclohexyl)methane, ethylenediamine, ethylenediamine carbamate, cyclohexanediamine, hexamethylenediamine benzoate, diamino-modified siloxane, 4,4'-methylenebiscyclohexylamine, bis(4-amino-3-methyldicyclohexyl)methane, 4,4'-methylenebiscyclohexylamine-cinnamaldehyde adduct, N,N'-dicinnamylidene-1,6-hexanediamine, 4,4'-($\alpha$,$\alpha$-dimethylbenzyl)diphenylamine, 4,4'-methylenedianiline, m-phenylenediamine, 4,4'-diaminodiphenyl ether, p-phenylenediamine, p,p'-ethylenedianiline, 4,4'-(p-phenylenediisopropylidene)dianiline, 4,4'-(m-phenylenediisopropylidene)dianiline, 3,4'-diaminodiphenyl ether, 4,4'-diaminodiphenyl sulfone, 2,2-bis[4-(4-aminophenoxy)phenyl]propane, bis[4-(4-aminophenoxy)phenyl]sulfone, bis[4-(3-aminophenoxy)phenyl]sulfone, 4,4'-bis(4-aminophenoxy)biphenol, bis[4-(4-aminophenoxy)phenyl]ether, 2,2-bis[4-(4-aminophenoxy)phenyl]hexafluoropropane, 1,4-bis(4-aminophenoxy)benzene, 1,3-bis(4-aminophenoxy)benzene, m-xylylenediamine, p-xylylenediamine, and the like.

In crosslinking with a polyvalent amine compound, a guanidine compound, a diazabicycloalkene compound, or an organic acid salt thereof, and the like, is used as a crosslinking accelerator.

Examples of guanidine compound include tetramethylguanidine, tetraethylguanidine, 1,3-diphenylguanidine, 1,3-di-o-tolylguanidine, and the like. Preferably 1,3-diphenylguanidine, 1,3-di-o-tolylguanidine, or a combination thereof.

The diazabicycloalkene compound is preferably 1,8-diazabicyclo[5.4.0]-7-undecene.

The organic acid salt of diazabicycloalkene compound is preferably an organic acid salt of 1,8-diazabicyclo[5.4.0]-7-undecene.

Examples of the organic acid used in the organic acid salt of 1,8-diazabicyclo[5.4.0]-7-undecane include organic monobasic acids or organic dibasic acids.

Examples of the organic monobasic acid include n-hexanoic acid, n-heptanoic acid, n-octanoic acid, 2-ethylhexanoic acid, n-capric acid, n-lauric acid, p-toluenesulfonic acid, phenol, and the like. Examples of the organic dibasic acid include adipic acid, pimelic acid, suberic acid, azelaic acid, sebacic acid, undecanedioic acid, dodecanedioic acid, terephthalic acid, orthophthalic acid, phthalic acid, and the like. Preferable examples are $C_{6-18}$ monocarboxylic acids or dicarboxylic acids.

When the crosslinking site monomer is an active chlorine-containing unsaturated monomer, a triazine thiol compound or a fatty acid alkali metal salt is used as the crosslinking agent.

Examples of triazine thiol compounds include triazine thiol (2,4,6-trimercapto-s-triazine) and a derivative thereof. Examples of derivatives include compounds in which part of the thiol group of triazine thiol is replaced by an amino group or an aliphatic hydrocarbon group. Preferred is 2,4,6-trimercapto-s-triazine.

When the crosslinking agent is a triazine thiol compound, a dithiocarbamate metal salt, thiuram sulfide, or the like can be preferably used as the crosslinking accelerator. Examples of the dithiocarbamate metal salt include zinc dimethyldithiocarbamate, zinc diethyldithiocarbamate, zinc di-n-butyldithiocarbamate, zinc di-n-hexyldithiocarbamate, zinc di-n-octyldithiocarbamate, zinc di-n-decyldithiocarbamate, zinc di-n-dodecyldithiocarbamate, zinc methylbenzyldithiocarbamate, zinc dibenzyldithiocarbamate, zinc methylcyclohexyldithiocarbamate, zinc dicyclohexyldithiocarbamate, and the like. Specific examples of the thiuram sulfide include tetramethylthiuram monosulfide, tetramethylthiuram disulfide, tetraethylthiuram disulfide, tetrabutylthiuram disulfide, dipentamethylenethiuram tetrasulfide, and the like.

Examples of fatty acid alkali metal salts include alkali metal salts of $C_{10-22}$ fatty acids. Particularly preferred are sodium stearate and potassium stearate.

Further, when a fatty acid alkali metal salt is used as the crosslinking agent, the crosslinking reaction can be effectively promoted by using sulfur in combination; thus, a crosslinking method using a fatty acid alkali metal salt and sulfur in combination is more common.

When the crosslinking site unsaturated monomer is an epoxy group-containing unsaturated monomer, an aromatic carboxylic acid ammonium salt, or a polyvalent amine compound that is used when the crosslinking site monomer is an $\alpha$,$\beta$-unsaturated carboxylic acid monomer, is used as the crosslinking agent. Examples of aromatic carboxylic acid ammonium salts include ammonium benzoate.

As the crosslinking accelerator that is used when the crosslinking site monomer is an epoxy group-containing unsaturated monomer, a crosslinking accelerator that is used when the crosslinking site monomer is an $\alpha$,$\beta$-unsaturated carboxylic acid monomer, an imidazole compound, a quaternary ammonium salt, a tertiary amine compound, an alkali metal salt of an aliphatic carboxylic acid, or the like can be used.

Examples of imidazole compounds include 2-methylimidazole, 2-phenylimidazole, and the like. Examples of quaternary ammonium salts include tetra-n-butylammonium bromide, octadecyl tri-n-butylammonium bromide, and the like. Examples of tertiary amine compounds include dimethylstearylamine. Examples of alkali metal salts of aliphatic carboxylic acids include sodium stearate, potassium stearate, and the like.

Crosslinking is carried out by primary crosslinking at about 120 to 250°C for about 1 to 60 minutes and optionally oven crosslinking (secondary crosslinking) at about 120 to 200°C for about 1 to 20 hours.

EXAMPLES

Next, the present invention will be described in detail with reference to Examples. The present invention, including its effects, is not limited to the Examples.

Reference Example 1

Production of compound (a)

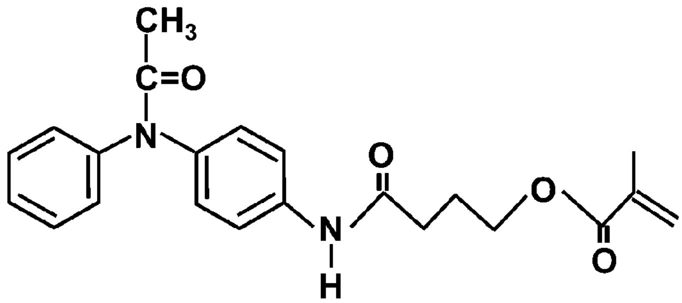

(a)

The compound (a) was produced by the following method.

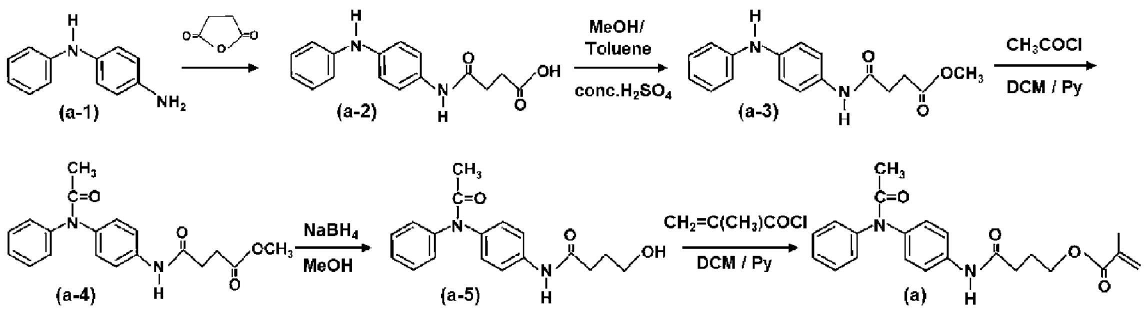


[First step] (a-1) -> (a-2):

In a 1000 ml three-necked flask equipped with a magnetic stirrer, a thermometer, and a dropping funnel, 36.0 g (359 mmol) of succinic anhydride, 30 g of N,N-dimethylacetamide, and 90 g of toluene were charged. A solution of 63.0 g (342 mmol) of 4-aminodiphenylamine dissolved in 120 g of toluene was added dropwise to this solution at room temperature over 1 hour, followed by further reaction for 0.5 hours. After the reaction was completed, 600 mL of toluene was added to the reaction mixture, and the produced solid was filtered off, thereby obtaining 87.1 g (crude yield: 90%) of a crude product as a brown solid. The obtained crude product was a two-component mixture containing a carboxylic acid compound (a-2) as a main component.

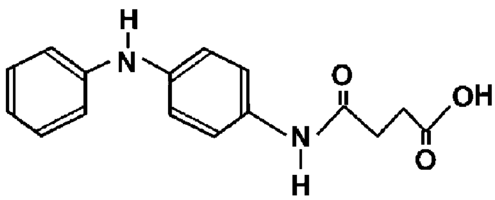

(a-2)

[Second step] (a-2) -> (a-3):

In a 2000-ml three-necked flask equipped with a magnetic stirrer, a thermometer, and a reflux cooling tube, 113.2 g (about 398 mmol) of the mixture of the carboxylic acid compound obtained in the first step above, 4.8 g of concentrated sulfuric acid, 96 g of methanol, and 1 L of toluene were charged and reacted at 70°C for 2 hours. After the reaction was completed, water and toluene were distilled off from the reaction mixture under reduced pressure, and the residue was then dissolved in methyl isobutyl ketone. The resultant was washed twice with a 1 wt.% aqueous sodium hydrogen carbonate solution, and anhydrous magnesium sulfate, synthetic aluminum silicate (Kyouwaad 700, produced by Kyowa Chemical Industry Co., Ltd.) as an alkali adsorbent, and activated carbon (Shirasagi A, produced by Osaka Gas Chemicals Co., Ltd.) were added to the organic layer. After the insoluble matter was filtered off, volatile components were distilled off under reduced pressure from the organic layer, thereby obtaining 115.5 g (crude yield: 97%) of a crude product as a pale red solid. Recrystallization of the obtained crude product was repeated three times using toluene containing Kyowaad 700, thereby obtaining 62.6 g (yield: 53%) of a carboxylic acid methyl ester (a-3) as a colorless solid.

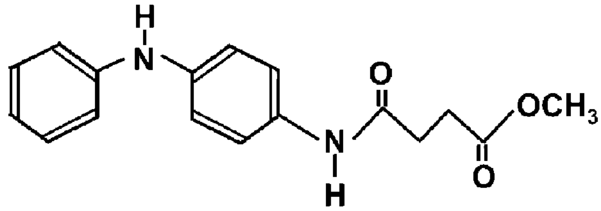

(a-3)

$^1H$ NMR(300MHz, Acetone-d6, $\delta$ ppm) :

2.65 (s, 4H, $-NHC(=O)CH_2CH_2C(=O)OCH_3$)
3.63 (s, 3H, $-NHC(=O)CH_2CH_2C(=O)OCH_3$)
6.79 (t, 1H, J=7.2Hz, Ar)
7.00-7.25 (m, 6H, Ar)
7.26 (brs, 1H, $-NHC(=O)CH_2CH_2C(=O)OCH_3$)
7.54 (d, 2H, J=9.0Hz, Ar)
9.05 (brs, 1H, ArNHAr)

[Third step] (a-3) -> (a-4):

In a 300 ml three-necked flask equipped with a magnetic stirrer, a thermometer, and a dropping funnel, 40 g (134 mmol) of the carboxylic acid methyl ester (a-3) obtained in the second step above, 17.8 g of pyridine, and 200 ml of dichloromethane were charged, and the content was cooled to 5°C. While keeping the internal temperature at 5 to 20°C, 13.8 g (176 mmol) of acetyl chloride was added dropwise, followed by further reaction for 1 hour. After the reaction was completed, 100 ml of dichloromethane was added for dilution, followed by washing three times with a saturated aqueous sodium chloride solution. The organic layer was dried over anhydrous magnesium sulfate, the insoluble matter was filtered off, and volatile components were then distilled off from the filtrate under reduced pressure, thereby obtaining 51.9 g of a crude product. The resultant was recrystallized using an ethyl acetate/n-hexane mixed solvent (volume ratio: 3/1), thereby obtaining 43.0 g (yield: 94%) of an N-acetylated methyl ester (a-4) as a colorless solid.

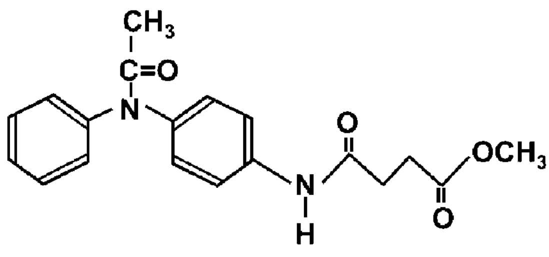

(a-4)

$^1H$ NMR(300MHz, Chloroform-d, $\delta$ ppm) :

2.06 (s, 3H, $N-C(=O)CH_3$)
2.64 (t, J=6.3Hz, 2H, $-NHC(=O)CH_2CH_2C(=O)OCH_3$)
2.74 (t, J=6.3Hz ,2H, $-NHC(=O)CH_2CH_2C(=O)OCH_3$)

3.71 (s, 3H, $-NHC(=O)CH_2CH_2C(=O)OCH_3$)
7.1-7.6 (m, 9H, Ar)
7.84 (brs, 1H, $-NHC(=O)CH_2CH_2C(=O)OCH_3$)

[Fourth step] (a-4) -> (a-5):

In a 1000 ml four-necked flask equipped with a magnetic stirrer, a thermometer, a gas outlet, and a reflux cooling tube, 30 g (88.1 mmol) of the N-acetylated methyl ester compound (a-4) obtained in the third step above and 150 ml of methanol were charged, and the content was heated to 45°C. While keeping the internal temperature at 45 to 55°C, 10 g (264 mmol) of sodium borohydride was slowly added, followed by further reaction for 1 hour. The methanol was distilled off under reduced pressure, and the residue was dissolved in methyl isobutyl ketone. The organic layer was washed twice with a saturated aqueous sodium chloride solution and then dried over anhydrous magnesium sulfate, after which the insoluble matter was filtered off. Volatile components were distilled off from the filtrate under reduced pressure to obtain 26.7 g (crude yield: 97%) of a terminal alcohol compound (a-5) as a colorless solid. Recrystallization was performed using an ethyl acetate/ethanol mixed solvent (volume ratio: 5/2), thereby obtaining 20.0 g (yield: 73%) of a terminal alcohol compound (a-5) as a colorless solid.

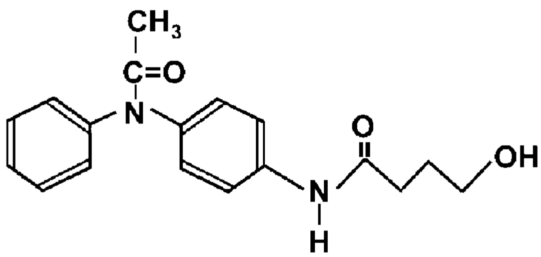

(a-5)

$^1H$ NMR(300MHz, Chloroform-d, $\delta$ ppm) :

1.91 (quin, J=6.3Hz, 2H, $-C(=O)C\underline{H}_2CH_2CH_2OH$)
2.06 (s, 3H, $N-C(=O)C\underline{H}_3$)
2.47 (t, J=6.6Hz, 2H, $-\underline{C}(=O)CH_2CH_2CH_2O-$)
2.65 (brs, 1H, $-C(=O)CH_2CH_2CH_2O\underline{H}$)
3.69 (t, J=6.0Hz, 2H, $-C(=O)CH_2CH_2C\underline{H}_2O-$)
7.1-7.6 (m, 9H, Ar)

[Fifth step] (a-5) -> (a):

In a 500 ml three-necked flask equipped with a magnetic stirrer, a thermometer, and a dropping funnel, 37.8 g (121 mmol) of the terminal alcohol compound (a-5) obtained in the fourth step above, 200 ml of dichloromethane, and 16.1 g of pyridine were charged, and the content was cooled to 5°C. While keeping the internal temperature at 5 to 15°C, 16.4 g (157 mmol) of methacryloyl chloride was added dropwise, followed by further reaction for 1 hour. 100 ml of dichloromethane was added to dilute the content, followed by washing three times with a saturated aqueous sodium chloride solution. The organic layer was dried over anhydrous magnesium sulfate, and the insoluble matter was filtered off, after which 200 ppm of 4-methoxyphenol based on the product was added to the filtrate. Volatile components were distilled off under reduced pressure to obtain 53.0 g (crude yield: 115%) of crude methacrylate (a). The crude methacrylate was subjected to column chromatography using ethyl acetate as the eluent (carrier: Wakogel C300), thereby obtaining 42.1 g (yield: 92%) of methacrylate (a) as a colorless viscous liquid.

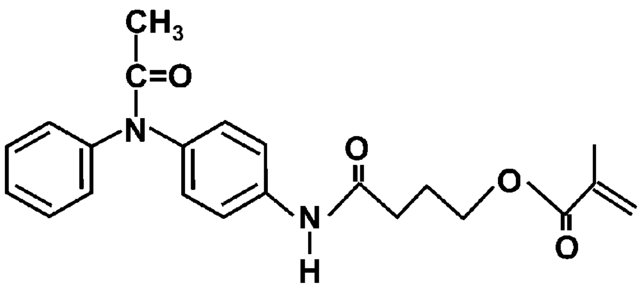

(a)

$^1$H NMR(300MHz, Cloroform-d, δ ppm) :

1.93 (t, J=1.2Hz, 3H, $CH_2=C-CH_3$)
2.06 (s, 3H, $N-C(=O)CH_3$)
2.09 (quin, J=6.6Hz, 2H, $-C(=O)CH_2CH_2CH_2O-$)
2.41 (t, J=7.2Hz, 2H, $-C(=O)CH_2CH_2CH_2O-$)
4.23 (t, J=6.0Hz, 2H, $-C(=O)CH_2CH_2CH_2O-$)
5.56 (t, J=1.5Hz, 1H, -C(=O)-C=C-H
(trans against the carbonyl group))
6.11 (s, 1H, -C(=O)-C=C-H
(cis against the carbonyl group))
6.1-7.8 (m, 9H, Ar)
8.00 (brs, 1H, NH)

Reference Example 2

Production of compound (b)

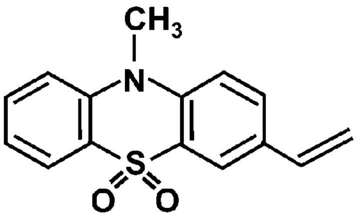

(b)

The compound (b) was produced by the following method.

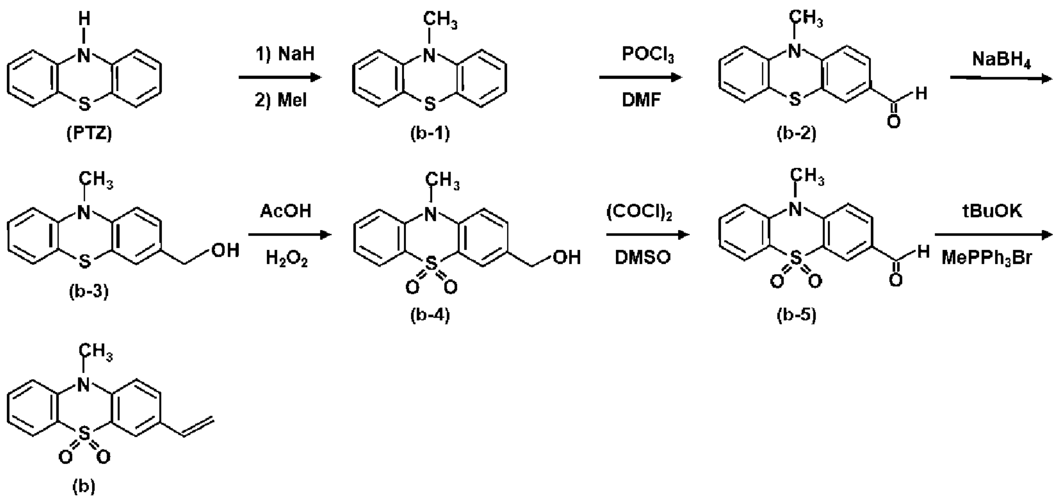

[First step] [PTZ] -> (b-1):

In a 500 ml four-necked flask equipped with a magnetic stirrer, a thermometer, a dropping funnel, and nitrogen gas inlet and outlet tubes, 40.0 g (201 mmol) of phenothiazine [PTZ] and 200 ml of N,N-dimethylformamide were charged, and the temperature in the system under nitrogen atmosphere was cooled to 5°C or less. While keeping the system temperature at 10°C or less, 7.2 g (300 mmol) of sodium hydride was added, followed by reaction for 1 hour. While keeping the system temperature at 20°C or less, 34.2 g (241 mmol) of iodomethane was added dropwise, followed by further reaction for 1 hour. After the reaction was completed, the reaction mixture was added to a saturated aqueous sodium chloride solution. The precipitated colorless solid was filtered off and dissolved in ethyl acetate. After the resultant was washed with a saturated aqueous sodium chloride solution, the organic layer was dried over anhydrous magnesium sulfate, and the insoluble matter was then filtered off. Volatile components were distilled off from the filtrate under reduced pressure to obtain 45.9 g (crude yield: 107%) of a crude product. Recrystallization was performed using ethanol, thereby obtaining 40.5 g (yield: 94%) of 10-methyl-10H-phenothiazine (b-1) as colorless needle crystals.

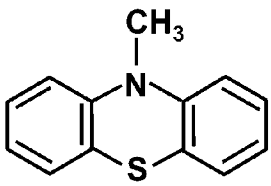

(b-1)

$^1H$ NMR(400MHz, Acetone-d6, $\delta$ ppm) :

3.39 (s, 3H, N-$CH_3$)
6.91-6.98 (m, 4H, Ar)
7.14 (dd, J=7.6Hz, J=1.6Hz, 2H, Ar)
7.21 (td, J=7.6Hz, J=1.6Hz, 2H, Ar)

[Second step] (b-1) -> (b-2):

In a 500 ml four-necked flask equipped with a magnetic stirrer, a dropping funnel, a thermometer, a gas inlet - a gas outlet, and a reflux cooling tube, 210 ml of N,N-dimethylformamide was charged. While keeping the internal temperature in the system at 10°C or less under nitrogen atmosphere, 129.4 g (844 mmol) of phosphoryl chloride was added dropwise, followed by further reaction for 30 minutes. Next, 30 g (141 mmol) of the N-methyl-10H-phenothiazine (b-1) obtained in the first step above was added, followed by reaction at 60°C for 24 hours. After the reaction was completed, the content was poured into an aqueous sodium acetate solution, and sodium hydrogen carbonate was further added for neutralization. The product was extracted from the obtained aqueous solution using ethyl acetate, and the organic layer was washed once with a saturated aqueous sodium chloride solution. The organic layer was dried over anhydrous magnesium sulfate, and the insoluble matter was filtered off, after which volatile components were distilled off from the filtrate under reduced pressure, thereby obtaining 33.5 g (crude yield: 98%) of a crude product as a red oily substance. Low-Rf components were removed from the crude product by column chromatography using ethyl acetate as the eluent (carrier: Wakogel C300), thereby obtaining 33.2 g (yield: 98%) of the target crude product as a yellow solid. Further, recrystallization was performed using ethyl acetate, thereby obtaining 30.1 g (yield: 88%) of 10-methyl-10H-phenothiazine-3-carbaldehyde (b-2) as yellow crystals.

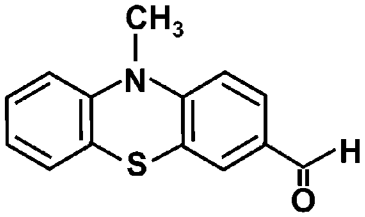

(b-2)

$^1H$ NMR(400MHz, Acetone d6, $\delta$ ppm) :

3.49 (s, 3H, N-$CH_3$)
7.00-7.06 (m, 2H, Ar)
7.10 (d, J=8.4Hz, 1H, Ar)

7.15-7.19(m, 1H, Ar)
7.22-7.28(m, 1H, Ar)
7.61(d, J=1.6Hz, 1H, Ar)
7.75(dd, J=8.4Hz, J=1.6Hz, 1H, Ar)
9.85(s, 1H, -CHO)

[Third step] (b-2) -> (b-3):

In a 2000 ml four-necked flask equipped with a magnetic stirrer, a thermometer, a gas outlet, and a reflux cooling tube, 30 g (124 mmol) of the compound (b-2) obtained in the second step above and 1200 ml of methanol were charged. While keeping the internal temperature at 30°C or less, 4.7 g (124 mmol) of sodium borohydride was slowly added, followed by further reaction for 1 hour. The methanol was distilled off under reduced pressure, and the residue was dissolved in methyl isobutyl ketone. The organic layer was washed once with a saturated aqueous sodium chloride solution, and after the washed organic layer was dried over anhydrous magnesium sulfate, the insoluble matter was filtered off. Volatile components were distilled off from the filtrate under reduced pressure to obtain 29.4 g (crude yield: 97%) of a crude product as a pale yellow solid. Recrystallization was performed using ethyl acetate, thereby obtaining 28.2 g (yield: 93%) of 3-hydroxymethyl-10-methyl-10H-phenothiazine (b-3) as a pale yellow solid.

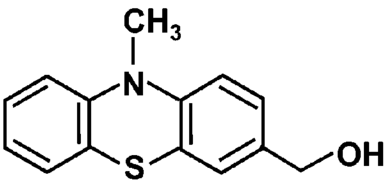

(b-3)

$^1$H NMR(400MHz, Acetone-d6, δ ppm) :

3.38 (s, 3H, N-C$H_3$)
4.08 (t, J=6.0Hz, 1H, -$CH_2$OH)
4.53 (d, J=6.0Hz, 2H, -C$H_2$OH)
6.87-6.98 (m, 3H, Ar)
7.10-7.23 (m, 4H, Ar)

[Fourth step] (b-3) -> (b-4):

In a 500 ml four-necked flask equipped with a magnetic stirrer, a thermometer, and a reflux cooling tube, 17.0 g (70 mmol) of the compound (b-3) obtained in the third step above, 150 ml of acetic acid, and 19.0 g (168 mmol) of a 30 wt.% hydrogen peroxide solution were charged and reacted at 60°C for 1 hour and then at 80°C for 1 hour. The acetic acid was distilled off from the reaction mixture under reduced pressure, and the residue was recrystallized using methanol, thereby obtaining 17.8 g (yield: 92%) of 3-hydroxymethyl-10-methyl-10H-phenothiazine-5,5-dioxide (b-4) as a pale yellow solid.

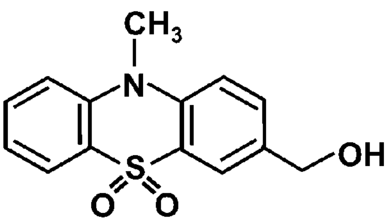

(b-4)

$^1$H NMR(400MHz, Acetone-d6, δ ppm) :

3.80 (s, 3H, -N-C$H_3$)
4.43 (t, J=6.0Hz, 1H, -$CH_2$OH)
4.75 (d, J=6.0Hz, 2H, -C$H_2$OH)
7.35 (t, J=7.6Hz, 1H, Ar)
7.56 (t, J=8.4Hz, 2H, Ar)
7.68-7.76 (m, 2H, Ar)

8.00-8.05 (m, 2H, Ar)

[Fifth step] (b-4) -> (b-5):

In a 500 ml four-necked flask equipped with a magnetic stirrer, a thermometer, a dropping funnel, a gas inlet tube, and a gas outlet tube, 15.3 g (121 mmol) of oxalyl chloride and 225 ml of dichloromethane were charged, and the internal temperature was cooled to -80 to -70°C under nitrogen atmosphere. 14.1 g (181 mmol) of dimethyl sulfoxide was slowly added dropwise, followed by further reaction for 30 minutes. Then, 16.5 g (60 mmol) of the compound (b-4) dissolved in 8 g of dimethyl sulfoxide in advance was added dropwise, followed by further reaction at -80 to -70°C for 2 hours. After 36.6 g of triethylamine was added, the internal temperature was raised to room temperature. A saturated aqueous sodium chloride solution was added to the reaction mixture, and the product was extracted with dichloromethane. After the organic layer was dried over anhydrous magnesium sulfate, the insoluble matter was filtered off, and volatile components were distilled off from the resulting filtrate under reduced pressure, thereby obtaining 28.0 g (crude yield: 94%) of a crude product. The crude product was subjected to column chromatography using dichloromethane as the eluent (carrier: Wakogel C300), thereby obtaining 15.4 g (yield: 94%) of a compound (b-5) as a pale yellow solid.

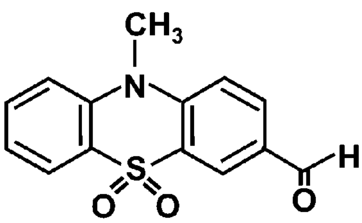

(b-5)

$^1$H NMR(400MHz, $CDCl_3$, δ ppm) :

3.79 (s, 3H, N-C<u>H</u>$_3$)
7.35-7.45 (m, 3H, Ar)
7.66-7.73 (m, 1H, Ar)
8.11-8.16 (m, 2H, Ar)
8.58 (d, J=1.6Hz, 1H, Ar)
10.00 (s, 1H, -C<u>H</u>O)

[Sixth step] (b-5) -> (b):

In a 500 ml four-necked flask equipped with a magnetic stirrer, a thermometer, a gas inlet tube, and a gas outlet tube, 250 ml of tetrahydrofuran was charged, and the internal temperature was cooled to 10°C or less while the inside of the reaction container was replaced with nitrogen. 11.2 g (99.7 mmol) of potassium tert-butoxide and then 35.6 g (99.7 mmol) of methyltriphenylphosphonium bromide were added, followed by reaction for 30 minutes. 22.7 g (83.1 mmol) of the compound (b-5) was added thereto, followed by reaction at 10 to 30°C for 2 hours. The obtained reaction mixture was added to a saturated aqueous sodium chloride solution, and the product was extracted with dichloromethane. After drying over anhydrous magnesium sulfate, the insoluble matter was filtered off, and volatile components were distilled off from the filtrate under reduced pressure to obtain 47.5 g of residue. The residue was subjected to column chromatography using dichloromethane as the eluent (carrier: Wakogel C300), and triphenylphosphine oxide was removed, followed by recrystallization using acetone, thereby obtaining 8.8 g (yield: 39%) of a compound (b) as a colorless solid.

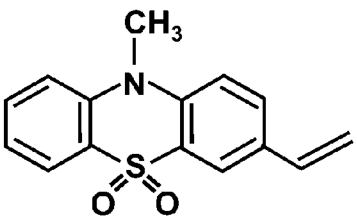

(b)

$^1$H NMR(400MHz, $CDCl_3$, δ ppm) :

3.71 (s, 3H, N-C<u>H</u>$_3$)

5.31 (d, J=11.2Hz, 1H, $C\underline{H}_2$=CH-Ar(trans against the Ar group))
5.79 (d, J=17.6Hz, 1H, $C\underline{H}_2$=CH-Ar(cis against the Ar group))
6.74 (dd, J=11.2Hz, 17.6Hz, 1H, $CH_2$=C$\underline{H}$-Ar)
7.23-7.34 (m, 3H, Ar)
7.59-7.69 (m, 2H, Ar)
8.09-8.15 (m, 2H, Ar)

Reference Example 3

Production of compound (c)

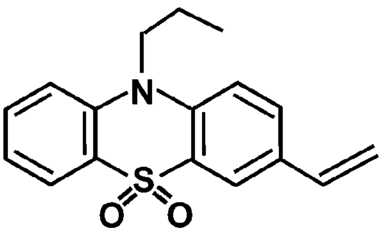

(c)

The compound (c) was produced by the following method.

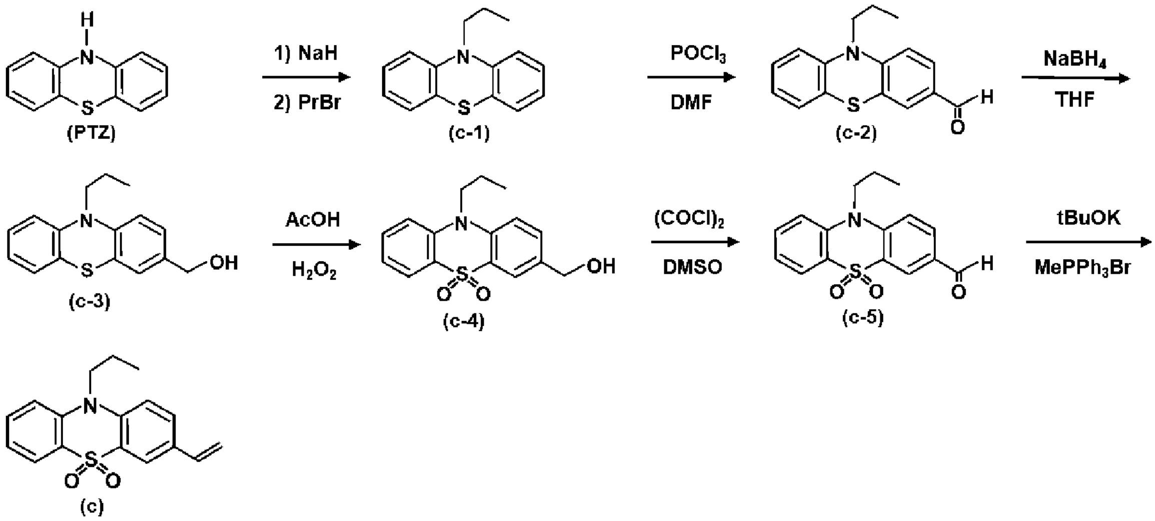


[First step] [PTZ] -> (c-1):

The same reaction as in the first step of Reference Example 1 was performed using 38.7 g (194 mmol) of phenothiazine [PTZ], 7.0 g (292 mmol) of sodium hydride, 28.6 g (233 mmol) of 1-bromopropane, and 200 ml of N,N-dimethylformamide. The obtained crude product was recrystallized using ethanol, thereby obtaining 42.1 g (yield: 90%) of a compound (c-1) as a colorless solid.

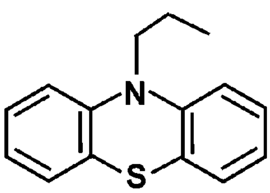

(c-1)

$^1$H NMR(400MHz, Acetone-d6, δ ppm) :

0.98 (t, J=7.2Hz, 3H, $-CH_2CH_2C\underline{H}_3$)
1.79 (sext, J=7.2Hz, 2H, $-CH_2C\underline{H}_2CH_3$)
3.90 (t, J=7.2Hz, 2H, $-C\underline{H}_2CH_2CH_3$)
6.93 (td, J=7.6, 1.2Hz, 2H, Ar)
7.00 (d, J=8.0Hz, 2H, Ar)
7.13 (dd, J=7.6Hz, 1.6Hz, 2H, Ar)
7.16-7.22 (m, 2H, Ar)

[Second step] (c-1) -> (c-2):

The same reaction as in the second step of Reference Example 1 was performed using 34 g (141 mmol) of the compound (c-1), 129.4 g (844 mmol) of phosphorus oxytrichloride, and 210 ml of N,N-dimethylformamide, thereby obtaining 35.8 g (yield: 94%) of a compound (c-2).

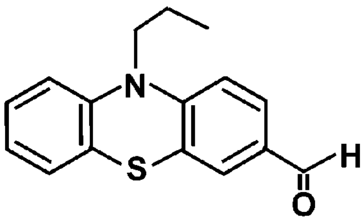

(c-2)

$^1H$ NMR (400MHz, Acetone-d6) δ ppm (TMS)

1.01 (t, J=7.2Hz, 3H, $NCH_2CH_2C\underline{H}_3$)
1.83 (sext, J=7.2Hz, 2H, $NCH_2C\underline{H}_2CH_3$)
3.99 (t, J=7.2Hz, 2H, $NC\underline{H}_2CH_2CH_3$)
7.01 (td, J=8.0Hz, 1.2Hz, 1H, Ar)
7.08 (d, J=7.6Hz, 1H, Ar)
7.12-7.18 (m, 2H, Ar)
7.20-7.26 (m, 1H, Ar)
7.60 (d, J=2.4Hz, 1H, Ar)
7.73 (dd, J=8.4Hz, 2.0Hz, 1H, Ar)
9.83 (s, 1H, $-C\underline{H}O$)

[Third step] (c-2) → (c-3):

The reaction was performed as in the third step of Reference Example 1 using 35.8 g (133 mmol) of the compound (c-2), 5.0 g (133 mmol) of a sodium borohydride, and 400 ml of tetrahydrofuran, followed by reaction at 60°C for 2 hours., thereby obtaining 36.1 g (yield: 100%) of a compound (c-3).

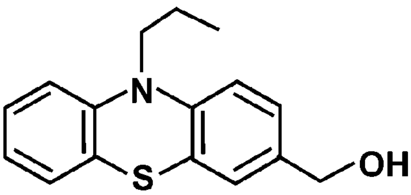

(c-3)

[Fourth step] (c-3) -> (c-4):

The reaction was performed as in the fourth step of Reference Example 1 using 36.1 g (133 mmol) of the compound (c-3), 45.6 g (402 mmol) of a 30 wt.% aqueous hydrogen peroxide solution, and 200 ml of acetic acid, thereby obtaining 40.6 g (yield:
100%) of a compound (c-4).

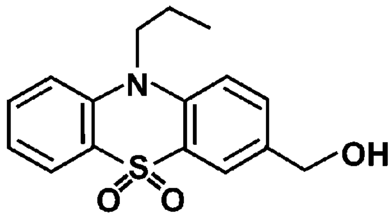

(c-4)

[Fifth step] (c-4) -> (c-5):

The reaction was performed as in the fifth step of Reference Example 1 using a solution prepared by dissolving 40.6 g (134 mmol) of the compound (c-4) in dimethyl sulfoxide (50 g), 33.9 g (268 mmol) of oxalyl chloride, 31.4 g (402 mmol) of dimethyl sulfoxide, 300 ml of dichloromethane acetic acid, and 81.2 g (804 mmol) of triethylamine, thereby obtaining 40.6 g (yield: 97%) of a compound (c-5).

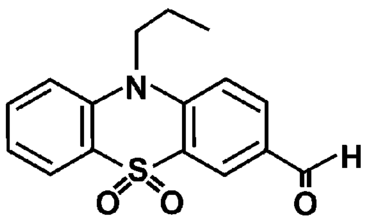

(c-5)

[Sixth step] (c-5) -> (c):

The reaction was performed as in the sixth step of Reference Example 1 using 39.2 g (130 mmol) of the compound (c-5), 17.5 g (156 mmol) of potassium tert-butoxide, 55.7 g (156 mmol) of methyltriphenylphosphonium bromide, and 350 ml of tetrahydrofuran, thereby obtaining 73.9 g of a crude product. The crude product was subjected to column chromatography using dichloromethane as the eluent (carrier: Wakogel C300), and triphenylphosphine oxide was removed, thereby obtaining 18.0 g (yield: 46%) of the target compound as a yellow solid. Further, recrystallization was performed using ethyl acetate, thereby obtaining 16.3 g (yield: 42%) of a compound (c).

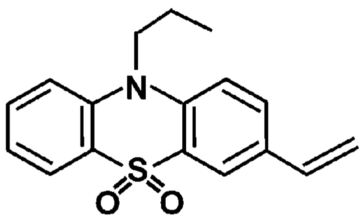

(c)

$^1$H NMR (400MHz, Chloroform-d) δ ppm (TMS)

1.07 (t, J=7.6Hz, 3H, $NCH_2CH_2C\underline{H}_3$)
1.95 (sext, J=7.6Hz, 2H, $NCH_2C\underline{H}_2CH_3$)
4.11 (t, J=7.6Hz, 2H, $NC\underline{H}_2CH_2CH_3$)
5.30 (d, J=11.2Hz, 1H, $C\underline{H}_2$=CH-Ar(trans against the Ar group))
5.78 (d, J=17.6Hz, 1H, $C\underline{H}_2$=CH-Ar(cis against the Ar group))
6.73 (dd, J=11.2Hz, 17.6Hz, 1H, $CH_2$=C$\underline{H}$-Ar)
7.23-7.35 (m, 3H, Ar)
7.58-7.68 (m, 2H, Ar)
8.10-8.16 (m, 2H, Ar)

Reference Example 4

The compound (d) was produced by the following method.

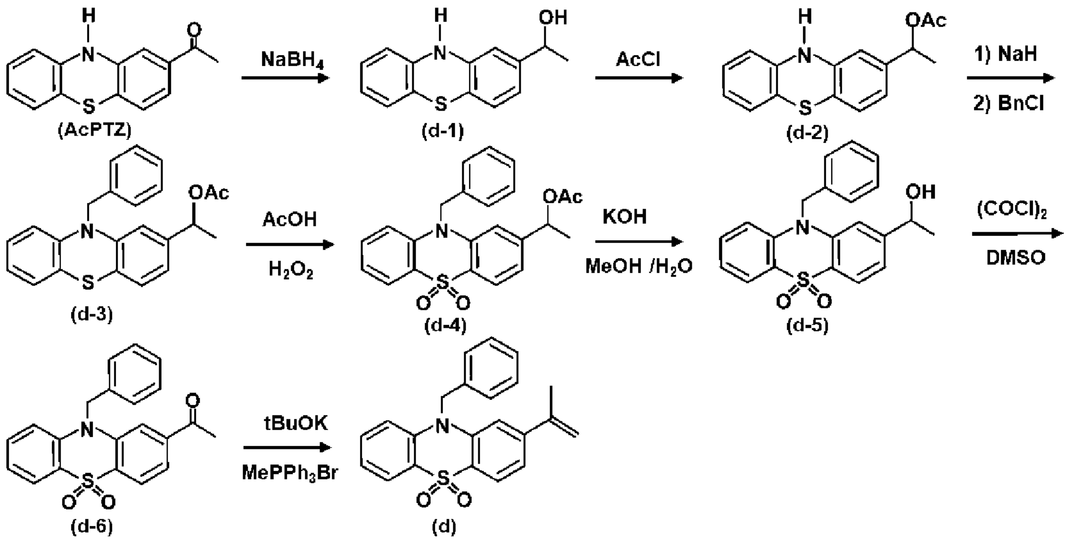


[First step] [AcPTZ] -> (d-1):

In a 2000 ml four-necked flask equipped with a magnetic stirrer, a thermometer, a gas outlet, and a reflux cooling tube, 25.0 g (104 mmol) of 2-acetylphenothiazine [AcPTZ] (produced by Tokyo Chemical Industry Co., Ltd.) and 1000 ml of methanol were charged. While keeping the internal temperature at 45 to 55°C, 19.6 g (518 mmol) of sodium borohydride was slowly added, followed by further reaction for 1 hour. The methanol was distilled off under reduced pressure, and the residue was dissolved in ethyl acetate. The organic layer was washed once with a saturated aqueous sodium chloride solution and then dried over anhydrous magnesium sulfate, after which the insoluble matter was filtered off. Volatile components were distilled off from the filtrate under reduced pressure to obtain 30.0 g of a crude product as a pale yellow solid. Recrystallization was performed using 85% ethanol, thereby obtaining 23.6 g (yield: 94%) of 2-(1-hydroxyethyl)-10H-phenothiazine (d-1) as a pale yellow solid.

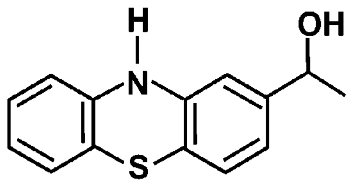

(d-1)

[Second step] (d-1) -> (d-2):

In a 500 ml four-necked flask equipped with a magnetic stirrer and a thermometer, 21.4 g (87.9 mmol) of the compound (d-1), 48.7 g (615 mmol) of pyridine, and 200 ml of dichloromethane were charged. While keeping the internal temperature at 15°C or less, 34.5 g (440 mmol) of acetyl chloride was added dropwise, followed by further reaction for 1 hour. The reaction mixture was added to a saturated aqueous sodium chloride solution, and the product was extracted with dichloromethane. Then, after drying over anhydrous magnesium sulfate, the insoluble matter was filtered off. Volatile components were distilled off from the filtrate under reduced pressure, thereby obtaining 23.7 g (yield: 94%) of 2-(1-acetoxyethyl)-10H-phenothiazine (d-2).

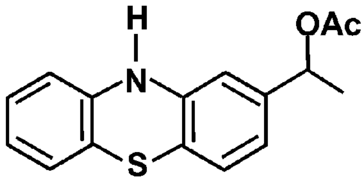

(d-2)

[Third step] (d-2) -> (d-3):

In a 500 ml four-necked flask equipped with a magnetic stirrer, a thermometer, a gas inlet, and a gas outlet,

23.7 g (83.0 mmol) of the compound (d-2) and 170 ml of N,N-dimethylformamide were charged. While keeping the internal temperature at 5°C or less, 3.0 g (125 mmol) of sodium hydride was added, followed by further reaction for 1 hour. Further, 14.7 g (116 mmol) of benzyl chloride was added, followed by reaction at 60°C for 1 hour. The reaction mixture was added to a saturated aqueous sodium chloride solution, and the product was extracted with ethyl acetate. Then, after drying over anhydrous magnesium sulfate, the insoluble matter was filtered off. Volatile components were distilled off from the filtrate under reduced pressure, thereby obtaining 34.9 g of crude 2-(1-acetoxyethyl)-10-benzyl-10H-phenothiazine (d-3).

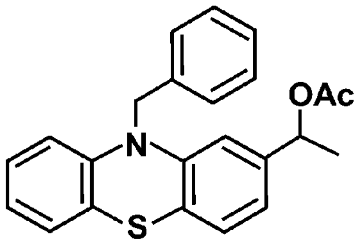

(d-3)

[Fourth step] (d-3) -> (d-4):

In a 500 ml four-necked flask equipped with a magnetic stirrer, a thermometer, and a reflux cooling tube, 34.9 g (about 83.0 mmol) of the compound (d-3), 50 g of acetic acid, 56 g (494 mmol) of 30 wt.% hydrogen peroxide, and 300 ml of toluene were charged. The reaction was performed at 50°C, 60°C, and 70°C each for 1 hour, and further at 80°C for 2 hours. The upper layer of the reaction mixture was removed, and volatile components were distilled off under reduced pressure to obtain 34.0 g of a crude product. Recrystallization was performed using a toluene/ethanol mixed solvent (volume ratio: 1/1), thereby obtaining 27.3 g (yield: 82% (based on the compound d-2)) of 2-(1-acetoxyethyl)-10-benzyl-10H-phenothiazine-5,5-dioxide (d-4).

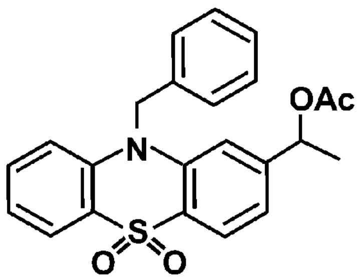

(d-4)

$^{1}H$ NMR (400MHz, Acetone-d6) δ ppm (TMS)

1.31 (d, J=6.8Hz, 3H, $\underline{CH_3}CH(OCOCH_3)$-Ar)
1.85 (s, 3H, $CH_3CH(OCO\underline{CH_3})$-Ar)
5.66 (s, 2H, Ar-$\underline{CH_2}$)
5.78 (q, J=6.8Hz, 1H, $CH_3C\underline{H}(OCOCH_3)$-Ar)
7.23-7.42 (m, 9H, Ar)
7.62 (t, J=8.4Hz, 1H, Ar)
8.04 (d, J=8.4Hz, 1H, Ar)
8.06 (dd, J=8.4Hz, 1H, Ar)

[Fifth step] (d-4) -> (d-5):

In a 1000 ml four-necked flask equipped with a magnetic stirrer, a thermometer, and a reflux cooling tube, 26.9 g (66.0 mmol) of the compound (d-4), 18.5 g (330 mmol) of potassium hydroxide, 500 ml of methanol, and 150 ml of water were charged, followed by reaction at 70°C for 2 hours. After the reaction was completed, volatile components were distilled off under reduced pressure, water was added to the residue, and the product was extracted with dichloromethane. Then, after drying over anhydrous magnesium sulfate, the insoluble matter was filtered off. Volatile components were distilled off from the filtrate under reduced pressure to obtain 23.4 g of a crude product. Recrystallization was performed using methanol, thereby obtaining 22.2 g (yield: 92%) of 2-(1-hydroxyethyl)-10-benzyl-10H-phenothiazine-5,5-dioxide (d-5) as a pale yellow solid.

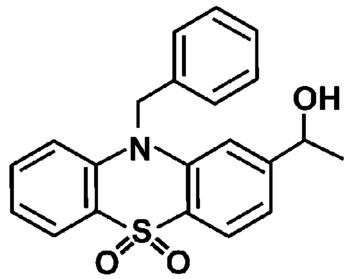

(d-5)

$^1$H NMR (400MHz, Acetone-d6) δ ppm (TMS)

1.31 (d, J=6.8Hz, 3H, $CH_3CH(OH)$-Ar)
2.82 (s, 1H, $CH_3CH(OH)$-Ar)
4.86 (quint, J=6.0Hz, 1H, $CH_3CH(OH)$-Ar)
5.63 (s, 2H, Ar-$CH_2$)
7.24-7.45 (m, 9H, Ar)
7.57-7.63 (m, 1H, Ar)
8.01 (d, J=8.0Hz, 1H, Ar)
8.07 (dd, J=8.4Hz, 2.0Hz, 1H, Ar)

[Sixth step] (d-5) -> (d-6):

In a 500 ml four-necked flask equipped with a magnetic stirrer, a thermometer, a gas inlet, and a gas outlet, 330 ml of dichloromethane and 14.4 g (113 mmol) of oxalyl chloride were charged. After the temperature in the reaction container was cooled to -70 to -80°C under nitrogen atmosphere, 13.3 g (170 mmol) of dimethyl sulfoxide was slowly added dropwise, and the same temperature was maintained for 1 hour. 20.7 g (56.7 mmol) of the compound (d-5) dissolved in 22 g of dimethyl sulfoxide in advance was added, followed by reaction for 2 hours at the same temperature. After 34 g (337 mmol) of triethylamine was slowly added, the temperature was slowly raised to room temperature, and the reaction mixture was added to a saturated aqueous sodium chloride solution. The product was extracted with dichloromethane, and then after drying over anhydrous magnesium sulfate, the insoluble matter was filtered off. Volatile components were distilled off from the filtrate under reduced pressure to obtain 29.2 g of a crude product. The crude product was subjected to column chromatography using dichloromethane as the eluent, thereby obtaining 17.8 g (yield: 86%) of 2-acetyl-10-benzyl-10H-phenothiazine-5,5-dioxide (d-6) as a pale yellow solid.

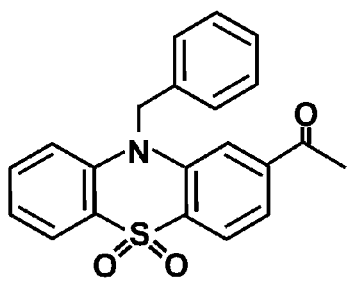

(d-6)

$^1$H NMR (400MHz, Chloroform-d) δ ppm (TMS)

2.50 (s, 3H, $CH_3(C=O)$-Ar)
5.48 (s, 2H, Ar-$CH_2$)
7.19 (d, J=8.8Hz, 3H, Ar)
7.28-7.40 (m, 4H, Ar)
7.52 (t,J=7.6Hz, 1H, Ar)
7.73 (s, 1H, Ar)
7.78 (d, J=8.4Hz, 1H, Ar)
8.15 (d, J=9.2Hz, 1H, Ar)
8.23 (d, J=8.4Hz, 1H, Ar)

[Seventh step] (d-6) → (d):

In a 500 ml four-necked flask equipped with a magnetic stirrer, a thermometer, a gas inlet, and a gas outlet, 200 ml of tetrahydrofuran was charged, and the internal temperature of the container was cooled to 0°C or less under nitrogen atmosphere. 8.2 g (73.5 mmol) of potassium tert-butoxide was added, the same temperature was maintained for 10 minutes, then 26.3 g (73.5 mmol) of methyltriphenylphosphonium bromide was added, and the same temperature was maintained for 30 minutes. 17.8 g (49.0 mmol) of the compound (d-6) was added thereto, followed by reaction at a temperature from 0°C or less to room temperature for 2 hours. The reaction mixture was added to a saturated aqueous sodium chloride solution, and the product was extracted with dichloromethane. Then, after drying over anhydrous magnesium sulfate, the insoluble matter was filtered off, and volatile components were distilled off from the filtrate under reduced pressure to obtain a crude product. The crude product was subjected to column chromatography using dichloromethane as the eluent (carrier: Wakogel C-300) and recrystallization using ethyl acetate, thereby obtaining 8.4 g (yield: 47%) of 2-(1-methylethenyl)-10-benzyl-10H-phenothiazine-5,5-dioxide (d) as a colorless solid.

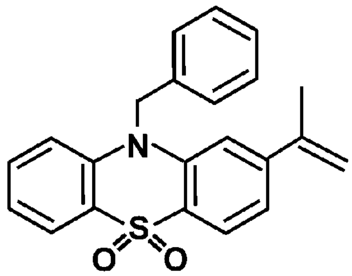

(d)

$^1$H NMR (400MHz, Chloroform-d) δ ppm (TMS)

1.96 (s, 3H, $CH_2$=$C\underline{H}(CH_3)$-Ar)
5.10 (s, 1H, $CH_2$=$C\underline{H}(CH_3)$-Ar(trans against the Ar group))
5.22 (s, 1H, $CH_2$=$C\underline{H}(CH_3)$-Ar(cis against the Ar group))
5.43(s, 2H, Ar-$CH_2$-)
7.12-7.42 (m, 9H, Ar)
7.49 (t, J=8.8Hz, 1H, Ar)
8.08 (d, J=8.4Hz, 1H, Ar)
8.15 (d, J=7.6Hz, 1H, Ar)

Reference Example 5

A compound (e) was produced from the compound (b-2) obtained through the same process as in Reference Example 2 by the following method.

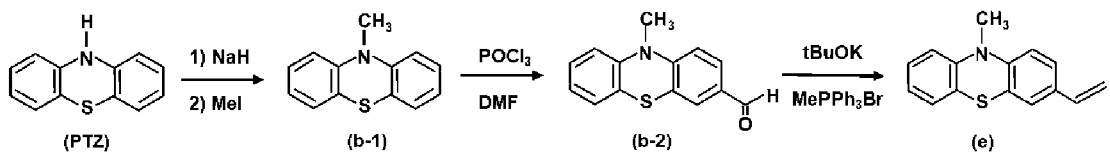


[Third step] (b-2) -> (e):

In a 500 ml four-necked flask equipped with a magnetic stirrer, a thermometer, a gas inlet tube, and a gas outlet tube, 220 ml of tetrahydrofuran was charged, and the internal temperature was cooled to 10°C or less while the inside of the reaction container was replaced with nitrogen. 8.4 g (74.9 mmol) of potassium tert-butoxide and then 26.9 g (75.3 mmol) of methyltriphenylphosphonium bromide were added, followed by reaction for 30 minutes. 15.0 g (62.2 mmol) of the compound (b-2) was added to the reaction mixture, followed by reaction at 10 to 30°C for 2 hours. The obtained reaction mixture was added to a saturated aqueous sodium chloride solution, and the product was extracted with dichloromethane. After drying over anhydrous magnesium sulfate, the insoluble matter was filtered off, and volatile components were distilled off from the filtrate under reduced pressure to obtain 35.1 g of residue. The residue was subjected to column chromatography using dichloromethane as the eluent (carrier: Wakogel C300), and triphenylphosphine oxide was removed, followed by recrystallization using ethanol, thereby obtaining 8.1 g (yield: 54%) of a compound (e) as a

pale yellow solid.

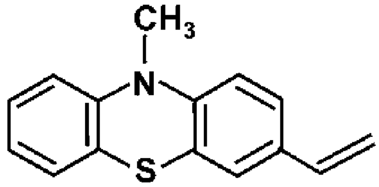

(e)

$^1H$ NMR(400MHz, $CDCl_3$, δ ppm) :

3.37 (s, 3H, N-$CH_3$)
5.14 (d, J=10.8Hz, 1H, C$\underline{H}_2$=CH-Ar(trans against the Ar group))
5.61 (d, J=17.6Hz, 1H, C$\underline{H}_2$=CH-Ar(cis against the Ar group))
6.59 (dd, J=10.8Hz, 17.6Hz, 1H, $CH_2$=C$\underline{H}$-Ar)
6.75 (d, J=8.4Hz, 1H, Ar)
6.81 (d, J=9.2Hz, 1H, Ar)
6.92 (td, J=7.6Hz, 1.2Hz, 1H, Ar)
7.11-7.23 (m, 4H, Ar)

Example 1

In a separable flask equipped with a thermometer, a stirrer, a nitrogen gas inlet tube, and a Dimroth cooling tube, the following components were charged.

| | |
|---|---|
| Water | 187 parts by weight |
| Sodium lauryl sulfate | 2 parts by weight |
| Polyoxyethylene lauryl ether | 2 parts by weight |

Charged monomer mixture

| | |
|---|---|
| Ethyl acrylate [EA] | 97.4 parts by weight |
| Mono n-butyl fumarate [MBF] | 1.6 parts by weight |
| Compound (a) of Reference Example 1 | 1.0 part by weight |

After oxygen was sufficiently removed from the system by replacement with nitrogen gas, the following components were added.

| | |
|---|---|
| Sodium formaldehyde sulfoxylate (Rongalite, produced by FUJIFILM Wako Pure Chemical Corporation) | 0.008 parts by weight |
| Tertiary butyl hydroperoxide (Perbutyl H69, produced by NOF Corporation) | 0.0047 parts by weight |

Then, a polymerization reaction was initiated at room temperature, and the reaction was continued until the polymerization conversion rate reached 90% or more. The obtained aqueous latex was coagulated with a 10 wt.% sodium sulfate aqueous solution, followed by water washing and drying, thereby obtaining an acrylic rubber A. The Mooney viscosity $PML_{1+4}$ (100°C) of the obtained acrylic rubber A was 32.

The molar fraction compositions of compound (a) and EA+MBF were 0.23 mol% and 99.77 mol% respectively, determined by $^1H$-NMR (400 MHz, Acetone-d6, δ ppm) using the following formulas.

α: integral value of signal at 7.0-8.2 ppm
β: integral value of signal at 3.2-5.0 ppm

$$\text{Compound (a) (mol\%)} = 200 \times \alpha/9\beta$$

$$EA+MBF\ (mol\%) = 100 - compound\ (a)$$

Moreover, the approximate weight fraction compositions of compound (a) and EA+MBF were 0.87 wt% and 99.13 wt%, respectively, determined by the following formulas.

$$Compound\ (a)\ (wt\%) = (compound\ (a)\ (mol\%) \times 380.3 \times 100)/ [compound\ (a)\ (mol\%) \times 380.3 + (EA+MBF\ (mol\%)) \times 100.8)]$$

$$EA+MBF\ (wt\%) = 100 - compound\ (a)\ (wt\%)$$

Example 2

A copolymerization reaction was performed in the same manner as in Example 1, except that the following charged monomer mixture was used, thereby obtaining acrylic rubber B. The Mooney viscosity $PML_{1+4}$ (100°C) of the obtained acrylic rubber B was 27.

Charged monomer mixture

| | |
|---|---|
| Ethyl acrylate [EA] | 97.4 parts by weight |
| Mono n-butyl fumarate [MBF] | 1.6 parts by weight |
| Compound (b) of Reference Example 2 | 1.0 part by weight |

The molar fraction compositions of compound (b) and EA+MBF were 0.36 mol% and 99.64 mol%, respectively, determined by $^1$H-NMR (400 MHz, Acetone-d6, δ ppm) using the following formulas.

α: integral value of signal at 7.0-8.2 ppm
β: integral value of signal at 3.2-5.0 ppm

$$Compound\ (b)\ (mol\%) = 200 \times \alpha/(2\alpha+7\beta)$$

$$EA+MBF\ (mol\%) = 100 - compound\ (b)$$

Moreover, the approximate weight fraction compositions of compound (b) and EA+MBF were 1.03 wt% and 98.97 wt%, respectively, determined by the following formulas.

$$Compound\ (b)\ (wt\%) = (compound\ (b)\ (mol\%) \times 271.34 \times 100)/ [compound\ (b)\ (mol\%) \times 271.34 + (EA+MBF\ (mol\%)) \times 100.8)]$$

$$EA+MBF\ (wt\%) = 100 - compound\ (b)\ (wt\%)$$

Example 3

A copolymerization reaction was performed in the same manner as in Example 1, except that the following charged monomer mixture was used, thereby obtaining acrylic rubber C. The Mooney viscosity $PML_{1+4}$ (100°C) of the obtained acrylic rubber C was 31.

Charged monomer mixture

| | |
|---|---|
| Ethyl acrylate [EA] | 97.4 parts by weight |
| Mono n-butyl fumarate [MBF] | 1.6 parts by weight |
| Compound (c) of Reference Example 3 | 1.0 part by weight |

The molar fraction compositions of compound (c) and EA+MBF were 0.34 mol% and 99.66 mol%, respectively, determined by $^1$H-NMR (400 MHz, Acetone-d6, $\delta$ ppm) using the following formulas.

$\alpha$: integral value of signal at 7.0-8.2 ppm
$\beta$: integral value of signal at 3.2-5.0 ppm

$$\text{Compound (c) (mol\%)} = 200 \times \alpha/(2\alpha+7\beta)$$

$$\text{EA+MBF (mol\%)} = 100 - \text{compound (b)}$$

Moreover, the approximate weight fraction compositions of compound (c) and EA+MBF were 1.01 wt% and 98.99 wt%, respectively, determined by the following formulas.

$$\text{Compound (c) (wt\%)} = (\text{compound (c) (mol\%)} \times 299.39 \times 100)/$$
$$[\text{compound (c) (mol\%)} \times 299.39 + (\text{EA+MBF (mol\%)}) \times 100.8)]$$

$$\text{EA+MBF (wt\%)} = 100 - \text{compound (c) (wt\%)}$$

Example 4

A copolymerization reaction was performed in the same manner as in Example 1, except that the following charged monomer mixture was used, thereby obtaining acrylic rubber D. The Mooney viscosity $PML_{1+4}$ (100°C) of the obtained acrylic rubber D was 33.

Charged monomer mixture

| | |
|---|---|
| Ethyl acrylate [EA] | 97.4 parts by weight |
| Mono n-butyl fumarate [MBF] | 1.6 parts by weight |
| Compound (d) of Reference Example 4 | 1.0 part by weight |

The molar fraction compositions of compound (d) and EA+MBF were 0.27 mol% and 99.73 mol%, respectively, determined by $^1$H-NMR (400 MHz, Acetone-d6, $\delta$ ppm) using the following formulas.

$\alpha$: integral value of signal at 7.0-8.2 ppm
$\beta$: integral value of signal at 3.2-5.0 ppm

$$\text{Compound (d) (mol\%)} = 200 \times \alpha/(\alpha+6\beta)$$

$$\text{EA+MBF (mol\%)} = 100 - \text{compound (d)}$$

Moreover, the approximate weight fraction compositions of compound (d) and EA+MBF were 0.96 wt% and 99.04 wt%, respectively, determined by the following formulas.

$$\text{Compound (d) (wt\%)} = (\text{compound (d) (mol\%)} \times 361.34 \times 100) / [\text{compound (d) (mol\%)} \times 361.34 + (\text{EA+MBF (mol\%)}) \times 100.8)]$$

$$\text{EA+MBF (wt\%)} = 100 - \text{compound (d) (wt\%)}$$

Reference Comparative Example 1

A copolymerization reaction was performed in the same manner as in Example 1, except that the following charged monomer mixture was used, thereby obtaining acrylic rubber E. The Mooney viscosity $PML_{1+4}$ (100°C) of the obtained acrylic rubber E was 32.

Charged monomer mixture

| | |
|---|---|
| Ethyl acrylate [EA] | 98.4 parts by weight |
| Mono n-butyl fumarate [MBF] | 1.6 parts by weight |

Example 5

| | |
|---|---|
| Acrylic rubber A | 100 parts by weight |
| FEF carbon black (Seast GSO, produced by Tokai Carbon Co., Ltd.) | 60 parts by weight |
| Stearic acid (TST, produced by Miyoshi Oil & Fat Co., Ltd.) | 1 part by weight |
| Polyoxyethylene stearyl ether phosphate (Phosphanol RL-210, produced by Toho Chemical Industry Co., Ltd.) | 0.5 parts by weight |
| Crosslinking accelerator (Vulcofac ACT55, produced by Safic-Alcan) | 1 part by weight |
| Hexamethylenediamine carbamate (Cheminox AC6F, produced by Unimatec Co., Ltd.) | 0.6 parts by weight |

Among the above components, the acrylic rubber A, FEF carbon black, stearic acid, and polyoxyethylene stearyl ether phosphate were mixed with a Banbury mixer. The obtained mixture and the other components were mixed using an open roll, thereby obtaining an acrylic rubber composition.

This composition was subjected to primary crosslinking at 180°C for 8 minutes and oven crosslinking (secondary crosslinking) at 175°C for 4 hours using a 100 ton press molding machine, thereby obtaining a sheet-like crosslinked product with a thickness of about 2 mm and a diameter of about 29 mm, and a cylindrical crosslinked product with a height of about 12.5 mm.

The crosslinking characteristics of the acrylic rubber composition and the physical properties of its crosslinked product were measured in the following manner.

Mooney scorch test: according to JIS K6300-1 (125°C).
Using a Mooney viscometer (AM-3, produced by Toyo Seiki Seisaku-sho, Ltd.), the minimum Mooney viscosity (ML min) and scorch time (t5) values were measured.
Crosslinking test: according to JIS K6300-2 (180°C, 12 minutes).

Using a rotorless rheometer (RLR-3, produced by Toyo Seiki Seisaku-sho, Ltd.), ML, MH, tc (10), and tc (90) values were measured.

ML: minimum torque
MH: maximum torque

tc (10): time required for the crosslinking torque to reach

$$ML + (MH - ML) x 0.1$$

tc (90): time required for the crosslinking torque to reach

$$ML + (MH - ML) x 0.9$$

Normal state physical properties: according to JIS K6251 and JIS K6253
Air heating aging test: according to JIS K6257

(190°C: 100 hours, 200 hours, 300 hours, 400 hours, 500 hours)
(175°C: 70 hours, 250 hours, 500 hours, 750 hours, 1000 hours)

Oil dipping test: After dipping in oil (IRM 903 oil) at 150°C for 168 hours according to JIS K6258, the hardness change compared to normal state physical properties before oil dipping, and other change rates and the volumetric swelling rate were determined for Example 6 and Comparative Examples 1 and 2.
Oil dipping-air heating aging combined test: After dipping in oil (IRM 903 oil) at 150°C for 168 hours according to JIS K6258, the oil component on the test piece was wiped off, an air heating aging test was performed at 190°C for 200 hours according to JIS K6257, and the normal state physical properties were compared before and after oil dipping.
Compression set test: according to JIS K6262 (175°C: 70 hours)

Example 6

In Example 5, the acrylic rubber B was used in place of the acrylic rubber A.

Example 7

In Example 5, the acrylic rubber C was used in place of the acrylic rubber A.

Example 8

In Example 5, the acrylic rubber D was used in place of the acrylic rubber A.

Comparative Example 1

In Example 5, the acrylic rubber E was used in place of the acrylic rubber A.

Comparative Example 2

In Comparative Example 1, 1.0 part by weight of 4,4'-bis(α,α-dimethylbenzyl)diphenylamine (Nocrac CD, produced by Ouchi Shinko Chemical Industrial Co., Ltd.) was further added.
Following Table 1 shows the results obtained in the above Examples 5 to 8 and Comparative Examples 1 to 2.

Table 1

| Measurement results | | Ex. 5 | Ex. 6 | Ex. 7 | Ex. 8 | Comp. Ex. 1 | Comp. Ex. 2 |
|---|---|---|---|---|---|---|---|
| Mooney scorch test (125°C) | | | | | | | |
| ML min | (pts) | 71 | 71 | 69 | 70 | 72 | 70 |
| t5 | (min) | 3.8 | 3.7 | 4.0 | 3.8 | 3.1 | 3.3 |
| Crosslinking test (180°C) | | | | | | | |
| tc (10) | (min) | 0.52 | 0.55 | 0.56 | 0.53 | 0.51 | 0.51 |

(continued)

| | | | | | | | |
|---|---|---|---|---|---|---|---|
| Crosslinking test (180°C) | | | | | | | |
| tc (90) | (min) | 5.62 | 5.67 | 5.50 | 5.55 | 5.26 | 5.19 |
| ML | (N•m) | 0.29 | 0.30 | 0.30 | 0.30 | 0.29 | 0.30 |
| MH | (N•m) | 0.96 | 1.00 | 0.99 | 1.04 | 1.08 | 1.08 |
| Normal state physical properties | | | | | | | |
| Hardness | (Duro A) | 68 | 71 | 71 | 72 | 71 | 69 |
| 100% modulus | (MPa) | 5.1 | 6.0 | 5.9 | 6.3 | 6.1 | 5.7 |
| Strength at break | (MPa) | 17.0 | 17.2 | 17.2 | 17.2 | 17.6 | 16.8 |
| Elongation at break | (%) | 260 | 240 | 250 | 230 | 230 | 230 |
| Air heating aging test (190°C, 100 hours) | | | | | | | |
| Hardness change | (Duro A) | +10 | +7 | +7 | +5 | +6 | +4 |
| Rate of change in 100% modulus | (%) | -21 | -32 | -39 | -40 | -48 | -40 |
| Rate of change in strength at break | (%) | -31 | -46 | -49 | -49 | -56 | -42 |
| Rate of change in elongation at break | (%) | +14 | +14 | +26 | +26 | +26 | +39 |
| Air heating aging test (190°C, 200 hours) | | | | | | | |
| Hardness change | (Duro A) | +9 | +8 | +7 | +5 | +8 | +4 |
| Rate of change in 100% modulus | (%) | -34 | -37 | -42 | -44 | -39 | -51 |
| Rate of change in strength at break | (%) | -54 | -61 | -65 | -65 | -66 | -64 |
| Rate of change in elongation at break | (%) | +13 | +5 | +13 | +19 | -39 | +32 |
| Air heating aging test (190°C, 300 hours) | | | | | | | |
| Hardness change | (Duro A) | +19 | +19 | +16 | +17 | +20 | +15 |
| Rate of change in 100% modulus | (%) | -8 | -3 | -10 | -11 | | -18 |
| Rate of change in strength at break | (%) | -67 | -63 | -67 | -65 | -58 | -68 |
| Rate of change in elongation at break | (%) | -26 | -37 | -39 | -43 | -78 | -21 |
| Air heating aging test (190°C, 400 hours) | | | | | | | |
| Hardness change | (Duro A) | +20 | +19 | +20 | +19 | +22 | +19 |
| Rate of change in 100% modulus | (%) | | | | | | |
| Rate of change in strength at break | (%) | -61 | -54 | -59 | -61 | -46 | -61 |
| Rate of change in elongation at break | (%) | -66 | -65 | -69 | -78 | -93 | -71 |
| Air heating aging test (190°C, 500 hours) | | | | | | | |
| Hardness change | (Duro A) | +29 | +26 | +25 | +23 | +28 | +24 |
| Rate of change in 100% modulus | (%) | | | | | | |
| Rate of change in strength at break | (%) | -36 | -21 | -24 | -24 | -26 | -28 |
| Rate of change in elongation at break | (%) | -90 | -91 | -92 | -94 | -99 | -93 |
| Air heating aging test (175°C, 70 hours) | | | | | | | |
| Hardness change | (Duro A) | +5 | +6 | +3 | +2 | +5 | +3 |

(continued)

| | | | | | | | |
|---|---|---|---|---|---|---|---|
| Air heating aging test (175°C, 70 hours) | | | | | | | |
| Rate of change in 100% modulus | (%) | -6 | -2 | -17 | -16 | -18 | -32 |
| Rate of change in strength at break | (%) | -9 | -12 | -16 | -12 | -17 | -18 |
| Rate of change in elongation at break | (%) | +5 | +4 | +8 | +13 | +7 | +17 |
| Air heating aging test (175°C, 250 hours) | | | | | | | |
| Hardness change | (Duro A) | +9 | +9 | +7 | +6 | +1 | +3 |
| Rate of change in 100% modulus | (%) | -10 | -23 | -32 | -37 | -39 | -35 |
| Rate of change in strength at break | (%) | -28 | -38 | -41 | -48 | -48 | -37 |
| Rate of change in elongation at break | (%) | +12 | +19 | +19 | +32 | +28 | +32 |
| Air heating aging test (175°C, 500 hours) | | | | | | | |
| Hardness change | (Duro A) | +16 | +13 | +13 | +12 | +13 | +9 |
| Rate of change in 100% modulus | (%) | -18 | -23 | -31 | -35 | -20 | -44 |
| Rate of change in strength at break | (%) | -54 | -59 | -64 | -63 | -65 | -65 |
| Rate of change in elongation at break | (%) | +10 | +7 | +11 | +18 | -9 | +40 |
| Air heating aging test (175°C, 750 hours) | | | | | | | |
| Hardness change | (Duro A) | +18 | +17 | -16 | +18 | +22 | +16 |
| Rate of change in 100% modulus | (%) | -6 | +0 | -7 | -10 | | -19 |
| Rate of change in strength at break | (%) | -62 | -60 | -63 | -62 | -51 | -65 |
| Rate of change in elongation at break | (%) | -4 | -20 | -15 | -12 | -59 | +8 |
| Air heating aging test (175°C, 1000 hours) | | | | | | | |
| Hardness change | (Duro A) | +25 | +25 | +23 | +25 | +27 | +25 |
| Rate of change in 100% modulus | (%) | | | | | | |
| Rate of change in strength at break | (%) | -53 | -30 | -35 | -33 | +31 | -46 |
| Rate of change in elongation at break | (%) | -70 | -73 | -73 | -75 | -96 | -77 |
| Oil dipping test (150°C, 168 hours) | | | | | | | |
| Hardness change | (Duro A) | - | -10 | - | - | -8 | -6 |
| Rate of change in 100% modulus | (%) | - | -8 | - | - | -2 | +3 |
| Rate of change in strength at break | (%) | - | -4 | - | - | -5 | -3 |
| Rate of change in elongation at break | (%) | - | -4 | - | - | -1 | -7 |
| Volumetric swelling rate | (%) | - | +11 | - | - | +11 | +10 |
| Oil dipping-air heating aging combined test | | | | | | | |
| Hardness change | (Duro A) | +15 | +15 | +12 | +15 | +17 | +17 |
| Rate of change in 100% modulus | (%) | -2 | -17 | -20 | -24 | +3 | +9 |

(continued)

| | | | | | | | |
|---|---|---|---|---|---|---|---|
| Oil dipping-air heating aging combined test | | | | | | | |
| Rate of change in strength at break | (%) | -51 | -59 | -61 | -62 | -59 | -58 |
| Rate of change in elongation at break | (%) | -8 | -12 | -7 | -6 | -42 | -41 |
| Compression set test (175°C, 70 hours) | (%) | 19 | 17 | 17 | 17 | 16 | 15 |

Example 9

A copolymerization reaction was performed in the same manner as in Example 1, except that the following charged monomer mixture was used, thereby obtaining acrylic rubber F. The Mooney viscosity $PML_{1+4}$ (100°C) of the obtained acrylic rubber F was 31.

Charged monomer mixture

| | |
|---|---|
| Ethyl acrylate [EA] | 97.4 parts by weight |
| Mono n-butyl fumarate [MBF] | 1.6 parts by weight |
| Compound (e) of Reference Example 5 | 1.0 part by weight |

The molar fraction compositions of compound (e) and EA+MBF were 0.41 mol% and 99.59 mol%, respectively, determined by $^1$H-NMR (400 MHz, Acetone-d6, $\delta$ ppm) using the following formulas.

$\alpha$: integral value of signal at 6.5-7.5 ppm
$\beta$: integral value of signal at 3.2-5.0 ppm

$$\text{Compound (e) (mol\%)} = 200 \text{ x } \alpha/(2\alpha+7\beta)$$

$$\text{EA+MBF (mol\%)} = 100 - \text{compound (e)}$$

Moreover, the approximate weight fraction compositions of compound (e) and EA+MBF were 1.0 wt% and 99.0 wt%, respectively, determined by the following formulas.

$$\text{Compound (e) (wt\%)} = (\text{compound (e) (mol\%)} \text{ x } 239.34 \text{ x } 100)/$$
$$[\text{compound (e) (mol\%)} \text{ x } 239.34 + (\text{EA+MBF (mol\%)}) \text{ x } 100.8)]$$

$$\text{EA+MBF (wt\%)} = 100 - \text{compound (e) (wt\%)}$$

Example 10

In Example 5, the SRF carbon black (Seast GS, produced by Tokai Carbon Co., Ltd.) was used in place of the FEF carbon black (Seast GSO), and the acrylic rubber F was used in place of the acrylic rubber A.

Comparative Example 3

In Example 10, the acrylic rubber E was used in place of the acrylic rubber F.

Comparative Example 4

In Comparative Example 3, 1.0 part by weight of 4,4'-bis($\alpha$,$\alpha$-dimethylbenzyl)diphenylamine (Nocrac CD) was further added.

The results obtained in Example 10 and Comparative Examples 3 and 4 above are shown in Table 2 below. For Example 10 and Comparative Examples 3 and 4, an air heating aging-oil dipping-air heating aging combined test was further performed under the following test conditions. Air heating aging-oil dipping-air heating aging combined test: An air heating aging test was performed at 175°C for 150 hours according to JIS K6257, an oil (IRM 903 oil) dipping test was performed at 150°C for 168 hours according to JIS K6258, the oil component on the test piece was wiped off, an air heating aging test was further performed at 190°C for 300 hours according to JIS K6257, and the normal state physical properties were compared before and after the test.

Table 2

| Measurement results | | | Ex. 10 | Comp. Ex. 3 | Comp. Ex. 4 |
|---|---|---|---|---|---|
| Mooney scorch test (125°C) | | | | | |
| | ML min | (pts) | 65 | 65 | 64 |
| | t5 | (min) | 1.5 | 2.8 | 2.4 |
| Crosslinking test (180°C) | | | | | |
| | tc (10) | (min) | 0.52 | 0.51 | 0.50 |
| | tc (90) | (min) | 5.43 | 4.93 | 4.87 |
| | ML | (N•m) | 0.24 | 0.22 | 0.23 |
| | MH | (N•m) | 0.96 | 0.98 | 0.97 |
| Normal state physical properties | | | | | |
| | Hardness | (Duro A) | 65 | 64 | 63 |
| | 100% modulus | (MPa) | 5.2 | 4.2 | 4.4 |
| | Strength at break | (MPa) | 17.1 | 17.1 | 15.8 |
| | Elongation at break | (%) | 260 | 270 | 260 |
| Air heating aging test (190°C, 100 hours) | | | | | |
| | Hardness change | (Duro A) | +9 | +3 | +0 |
| | Rate of change in 100% modulus | (%) | +62 | -48 | -36 |
| | Rate of change in strength at break | (%) | -8 | -65 | -35 |
| | Rate of change in elongation at break | (%) | -30 | +19 | +28 |
| Air heating aging test (190°C, 200 hours) | | | | | |
| | Hardness change | (Duro A) | +10 | +12 | +0 |
| | Rate of change in 100% modulus | (%) | +37 | -24 | -52 |
| | Rate of change in strength at break | (%) | -23 | -68 | -60 |
| | Rate of change in elongation at break | (%) | -30 | -30 | +31 |
| Air heating aging test (190°C, 300 hours) | | | | | |
| | Hardness change | (Duro A) | +13 | +20 | +3 |
| | Rate of change in 100% modulus | (%) | +17 | +26 | -48 |
| | Rate of change in strength at break | (%) | -41 | -68 | -70 |
| | Rate of change in elongation at break | (%) | -29 | -61 | +33 |
| Air heating aging test (190°C, 400 hours) | | | | | |
| | Hardness change | (Duro A) | +16 | +28 | +15 |
| | Rate of change in 100% modulus | (%) | +8 | | -25 |
| | Rate of change in strength at break | (%) | -57 | -56 | -69 |
| | Rate of change in elongation at break | (%) | -42 | -86 | -11 |
| Air heating aging test (190°C, 500 hours) | | | | | |
| | Hardness change | (Duro A) | +24 | +31 | +23 |
| | Rate of change in 100% modulus | (%) | +31 | | +14 |
| | Rate of change in strength at break | (%) | -58 | -31 | -65 |
| | Rate of change in elongation at break | (%) | -61 | -93 | -51 |

(continued)

| | | | | |
|---|---|---|---|---|
| Oil dipping test (150°C, 168 hours) | | | | |
| Hardness change | (Duro A) | -6 | -7 | -7 |
| Rate of change in 100% modulus | (%) | -6 | +12 | +5 |
| Rate of change in strength at break | (%) | -10 | -9 | -6 |
| Rate of change in elongation at break | (%) | -4 | -9 | -6 |
| Volumetric swelling rate | (%) | +11 | +11 | +10 |
| Oil dipping-air heating aging combined test | | | | |
| Hardness change | (Duro A) | +13 | +13 | +12 |
| Rate of change in 100% modulus | (%) | +38 | -12 | -20 |
| Rate of change in strength at break | (%) | -30 | -65 | -61 |
| Rate of change in elongation at break | (%) | -37 | -31 | -18 |
| Air heating aging-oil dipping-air heating aging combined test | | | | |
| Hardness change | (Duro A) | +17 | +23 | +19 |
| Rate of change in 100% modulus | (%) | +15 | | +9 |
| Rate of change in strength at break | (%) | -51 | -61 | -68 |
| Rate of change in elongation at break | (%) | -39 | -71 | -60 |
| Compression set test (175°C, 70 hours) | (%) | 14 | 14 | 14 |

Example 11

A copolymerization reaction was performed in the same manner as in Example 1, except that the following charged monomer mixture was used, thereby obtaining acrylic rubber G. The Mooney viscosity $PML_{1+4}$ (100°C) of the obtained acrylic rubber G was 30.

Charged monomer mixture

| | |
|---|---|
| Ethyl acrylate [EA] | 97.9 parts by weight |
| Mono n-butyl fumarate [MBF] | 1.6 parts by weight |
| Compound (e) of Reference Example 5 | 0.5 parts by weight |

The mole fraction compositions were as follows: compound (e): 0.20 mol%, EA+MBF: 99.80 mol%.

Further, the approximate weight fraction compositions were as follows: compound (e): 0.48 wt.%, EA+MBF: 99.52 wt.%.

Example 12

In Example 5, the following component was used.

| | |
|---|---|
| Acrylic rubber G | 100 parts by weight |
| SRF carbon black (Seast GS) | 70 parts by weight |
| Stearic acid (TST) | 1 part by weight |
| Polyoxyethylene stearyl ether phosphate (Phosphanol RL-210) | 0.5 parts by weight |
| Stearyl amine (Farmin 80S, produced by Kao Corporation) | 1 part by weight |
| Crosslinking accelerator (Vulcofac ACT55) | 1 part by weight |
| Hexamethylenediamine carbamate (Cheminox AC6F) | 0.6 parts by weight |

Comparative Example 5

In Example 12, the acrylic rubber E was used in place of the acrylic rubber G.

Comparative Example 6

In Comparative Example 5, 0.5 parts by weight of the compound (b-1) produced in Reference Example 5 was further used.

Comparative Example 7

In Comparative Example 5, 0.5 parts by weight of the compound (e) was further used.
Following Table 3 shows the results obtained in the above Example 12 and Comparative Examples 5 to 7.

Table 3

| Measurement results | | | Ex. 12 | Comp. Ex. 5 | Comp. Ex. 6 | Comp. Ex. 7 |
|---|---|---|---|---|---|---|
| Mooney scorch test (125°C) | | | | | | |
| | ML min | (pts) | 59 | 61 | 58 | 58 |
| | t5 | (min) | 4.2 | 3.8 | 4.0 | 4.0 |
| Crosslinking test (180°C) | | | | | | |
| | tc (10) | (min) | 0.58 | 0.57 | 0.57 | 0.56 |
| | tc (90) | (min) | 6.62 | 6.58 | 6.54 | 6.54 |
| | ML | (N•m) | 0.26 | 0.26 | 0.26 | 0.26 |
| | MH | (N•m) | 0.95 | 1.00 | 0.99 | 0.95 |
| Normal state physical properties | | | | | | |
| | Hardness | (Duro A) | 70 | 70 | 69 | 70 |
| | 100% modulus | (MPa) | 5.1 | 5.6 | 6.0 | 5.5 |
| | Strength at break | (MPa) | 15.5 | 15.3 | 15.4 | 14.6 |
| | Elongation at break | (%) | 270 | 240 | 240 | 260 |
| Air heating aging test (190°C, 100 hours) | | | | | | |
| | Hardness change | (Duro A) | +10 | +7 | +6 | +9 |
| | Rate of change in 100% modulus | (%) | +27 | -38 | -42 | -24 |
| | Rate of change in strength at break | (%) | -9 | -47 | -49 | -29 |
| | Rate of change in elongation at break | (%) | -20 | +7 | +6 | -1 |
| Air heating aging test (190°C, 200 hours) | | | | | | |
| | Hardness change | (Duro A) | +9 | +9 | +11 | +10 |
| | Rate of change in 100% modulus | (%) | +10 | -29 | -37 | -38 |
| | Rate of change in strength at break | (%) | -25 | -59 | -59 | -58 |
| | Rate of change in elongation at break | (%) | -14 | -10 | -9 | -3 |
| Air heating aging test (190°C, 300 hours) | | | | | | |
| | Hardness change | (Duro A) | +15 | +20 | +23 | +21 |
| | Rate of change in 100% modulus | (%) | -2 | | | +7 |
| | Rate of change in strength at break | (%) | -49 | -56 | -55 | -57 |
| | Rate of change in elongation at break | (%) | -22 | -66 | -62 | -55 |
| Air heating aging test (190°C, 400 hours) | | | | | | |
| | Hardness change | (Duro A) | +17 | +25 | +24 | +23 |
| | Rate of change in 100% modulus | (%) | +10 | | | |
| | Rate of change in strength at break | (%) | -54 | -29 | -32 | -37 |
| | Rate of change in elongation at break | (%) | -37 | -86 | -86 | -82 |
| Air heating aging test (190°C, 500 hours) | | | | | | |
| | Hardness change | (Duro A) | +21 | +25 | +28 | +24 |
| | Rate of change in 100% modulus | (%) | +41 | | | |

(continued)

| | | | | | | |
|---|---|---|---|---|---|---|
| Air heating aging test (190°C, 500 hours) | | | | | | |
| | Rate of change in strength at break | (%) | -52 | +11 | +5 | +0 |
| | Rate of change in elongation at break | (%) | -60 | -96 | -96 | -95 |
| Air heating aging test (190°C, 600 hours) | | | | | | |
| | Hardness change | (Duro A) | +24 | +29 | +28 | +27 |
| | Rate of change in 100% modulus | (%) | | | | |
| | Rate of change in strength at break | (%) | -11 | -7 | -16 | -32 |
| | Rate of change in elongation at break | (%) | -92 | -100 | -100 | -100 |
| Compression set test | | | | | | |
| | 175°C, 70 hours | (%) | 21 | 20 | 20 | 21 |
| | 175°C, 500 hours | (%) | 38 | 36 | 36 | 35 |

Example 13

A copolymerization reaction was performed in the same manner as in Example 1, except that the following charged monomer mixture was used, thereby obtaining acrylic rubber H. The Mooney viscosity $PML_{1+4}$ (100°C) of the obtained acrylic rubber H was 38.

Charged monomer mixture

| | |
|---|---|
| Ethyl acrylate [EA] | 97.0 parts by weight |
| Vinyl chloroacetate [VCA] | 2.5 parts by weight |
| Compound (e) of Reference Example 5 | 0.5 parts by weight |

The mole fraction compositions were as follows: compound (e): 0.22 mol%, EA+VCA: 99.78 mol%.
Further, the approximate weight fraction compositions were as follows: compound (e): 0.51 wt.%, EA+VCA: 99.49 wt.%.

Reference Comparative Example 2

A copolymerization reaction was performed in the same manner as in Example 1, except that the following charged monomer mixture was used, thereby obtaining acrylic rubber J. The Mooney viscosity $PML_{1+4}$ (100°C) of the obtained acrylic rubber J was 43.

Charged monomer mixture

| | |
|---|---|
| Ethyl acrylate [EA] | 97.5 parts by weight |
| Vinyl chloroacetate [VCA] | 2.5 parts by weight |

Example 14

In Example 5, the following component was used.

| | |
|---|---|
| Acrylic rubber H | 100 parts by weight |
| FEF carbon black (Seast GSO) | 60 parts by weight |
| Stearic acid (TST) | 1 part by weight |
| Fatty acid sodium (Na-soap, produced by Kao Chemical Corporation) | 3 parts by weight |

(continued)

| | |
|---|---|
| Fatty acid potassium (Nonsoul SK-1,produced by NOF Corporation) | 0.25 parts by weight |
| Sulfur (precipitated sulfur, produced by Hosoi Chemical Industry Co., Ltd.) | 0.3 parts by weight |

Comparative Example 8

In Example 14, the acrylic rubber J was used in place of the acrylic rubber H.

Comparative Example 9

In Comparative Example 8, 1.0 part by weight of 4,4'-bis(α,α-dimethylbenzyl)diphenylamine (Nocrac CD) was further added.

Example 15

In Example 5, the following component was used.

| | |
|---|---|
| Acrylic rubber H | 100 parts by weight |
| FEF carbon black (Seast GSO) | 60 parts by weight |
| Stearic acid (TST) | 1 part by weight |
| 2,4,6-trimercapto-s-triazine (Nocceler TCA, produced by Ouchi Shinko Chemical Industrial Co., Ltd.) | 0.5 parts by weight |
| Zinc dibutyldithiocarbamate (Nocceler BZ, produced by Ouchi Shinko Chemical Industrial Co., Ltd.) | 1.5 parts by weight |

Comparative Example 10

In Example 15, the acrylic rubber J was used in place of the acrylic rubber H.

Comparative Example 11

In Comparative Example 10, 1.0 part by weight of 4,4'-bis(α,α-dimethylbenzyl)diphenylamine (Nocrac CD) was further added.

Following Table 4 shows the results obtained in the above Examples 14 to 15 and Comparative Examples 8 to 11.

Table 4

| Measurement results | | Ex. 14 | Comp. Ex. 8 | Comp. Ex. 9 | Ex. 15 | Comp. Ex. 10 | Comp. Ex. 11 |
|---|---|---|---|---|---|---|---|
| Mooney scorch test (125°C) | | | | | | | |
| ML min | (pts) | 67 | 71 | 69 | 60 | 59 | 60 |
| t5 | (min) | 7.6 | 8.0 | 7.3 | 11.1 | 7.3 | 9.4 |
| Crosslinking test (180°C) | | | | | | | |
| tc (10) | (min) | 0.88 | 0.92 | 0.93 | 1.54 | 1.46 | 1.46 |
| tc (90) | (min) | 4.82 | 4.44 | 4.43 | 6.64 | 6.30 | 6.22 |
| ML | (N•m) | 0.29 | 0.30 | 0.30 | 0.27 | 0.28 | 0.26 |
| MH | (N•m) | 1.14 | 1.21 | 1.19 | 1.18 | 1.30 | 1.24 |
| Normal state physical properties | | | | | | | |
| Hardness | (Duro A) | 67 | 67 | 66 | 68 | 69 | 69 |
| 100% modulus | (MPa) | 5.8 | 6.2 | 6.4 | 6.3 | 7.0 | 7.0 |
| Strength at break | (MPa) | 16.5 | 15.9 | 15.6 | 14.7 | 14.8 | 14.6 |
| Elongation at break | (%) | 280 | 280 | 280 | 280 | 260 | 240 |

(continued)

| | | | | | | | |
|---|---|---|---|---|---|---|---|
| Air heating aging test (150°C, 70 hours) | | | | | | | |
| Hardness change | (Duro A) | +4 | +6 | +6 | +7 | +6 | +6 |
| Rate of change in 100% modulus | (%) | +14 | -13 | -17 | +14 | +1 | -11 |
| Rate of change in strength at break | (%) | +7 | -3 | -6 | +14 | +1 | -3 |
| Rate of change in elongation at break | (%) | -10 | -7 | +0 | -18 | -9 | +12 |
| Air heating aging test (150°C, 200 hours) | | | | | | | |
| Hardness change | (Duro A) | +4 | +4 | +5 | +6 | +6 | +5 |
| Rate of change in 100% modulus | (%) | +14 | -31 | -30 | +19 | +4 | -14 |
| Rate of change in strength at break | (%) | +8 | -15 | -7 | +17 | +10 | +0 |
| Rate of change in elongation at break | (%) | -10 | +1 | +13 | -21 | -7 | +12 |
| Air heating aging test (150°C, 400 hours) | | | | | | | |
| Hardness change | (Duro A) | +19 | +19 | +16 | +17 | +20 | +15 |
| Rate of change in 100% modulus | (%) | -8 | -3 | -10 | -11 | | -18 |
| Rate of change in strength at break | (%) | -67 | -63 | -67 | -65 | -58 | -68 |
| Rate of change in elongation at break | (%) | -26 | -37 | -39 | -43 | -78 | -21 |
| Air heating aging test (150°C, 600 hours) | | | | | | | |
| Hardness change | (Duro A) | +20 | +19 | +20 | +19 | +22 | +19 |
| Rate of change in 100% modulus | (%) | | | | | | |
| Rate of change in strength at break | (%) | -61 | -54 | -59 | -61 | -46 | -61 |
| Rate of change in elongation at break | (%) | -66 | -65 | -69 | -78 | -93 | -71 |
| Air heating aging test (150°C, 800 hours) | | | | | | | |
| Hardness change | (Duro A) | +29 | +26 | +25 | +23 | +28 | +24 |
| Rate of change in 100% modulus | (%) | | | | | | |
| Rate of change in strength at break | (%) | -36 | -21 | -24 | -24 | -26 | -28 |
| Rate of change in elongation at break | (%) | -90 | -91 | -92 | -94 | -99 | -93 |
| Air heating aging test (150°C, 1000 hours) | | | | | | | |
| Hardness change | (Duro A) | +5 | +6 | +3 | +2 | +5 | +3 |
| Rate of change in 100% modulus | (%) | -6 | -2 | -17 | -16 | -18 | -32 |
| Rate of change in strength at break | (%) | -9 | -12 | -16 | -12 | -17 | -18 |
| Rate of change in elongation at break | (%) | +5 | +4 | +8 | +13 | +7 | +17 |
| Air heating aging test (175°C, 100 hours) | | | | | | | |
| Hardness change | (Duro A) | +5 | +2 | +6 | +9 | +6 | +7 |

(continued)

| | | | | | | | |
|---|---|---|---|---|---|---|---|
| Air heating aging test (175°C, 100 hours) | | | | | | | |
| Rate of change in 100% modulus | (%) | -17 | -65 | -50 | +3 | -37 | -27 |
| Rate of change in strength at break | (%) | -14 | -78 | -34 | +9 | -26 | -10 |
| Rate of change in elongation at break | (%) | +0 | +30 | +30 | -13 | -5 | +16 |
| Air heating aging test (175°C, 200 hours) | | | | | | | |
| Hardness change | (Duro A) | +6 | +11 | +5 | +9 | +10 | +9 |
| Rate of change in 100% modulus | (%) | -36 | -60 | -67 | -11 | -60 | -43 |
| Rate of change in strength at break | (%) | -40 | -79 | -70 | -10 | -68 | -29 |
| Rate of change in elongation at break | (%) | +17 | +0 | +78 | -9 | +18 | +26 |
| Air heating aging test (175°C, 300 hours) | | | | | | | |
| Hardness change | (Duro A) | +8 | +18 | +8 | +14 | +16 | +9 |
| Rate of change in 100% modulus | (%) | -48 | -29 | -75 | -29 | -61 | -60 |
| Rate of change in strength at break | (%) | -58 | -72 | -84 | -33 | -82 | -58 |
| Rate of change in elongation at break | (%) | +25 | -63 | +86 | -5 | -6 | +41 |
| Air heating aging test (175°C, 400 hours) | | | | | | | |
| Hardness change | (Duro A) | +10 | +23 | +6 | +13 | +20 | +8 |
| Rate of change in 100% modulus | (%) | -57 | | -73 | -41 | | -69 |
| Rate of change in strength at break | (%) | -68 | -66 | -88 | -48 | -76 | -77 |
| Rate of change in elongation at break | (%) | +28 | -82 | +70 | +0 | -64 | +78 |
| Air heating aging test (175°C, 500 hours) | | | | | | | |
| Hardness change | (Duro A) | +12 | +28 | +13 | +16 | +25 | +14 |
| Rate of change in 100% modulus | (%) | -55 | | -67 | -49 | | -71 |
| Rate of change in strength at break | (%) | -75 | -45 | -85 | -66 | +59 | -86 |
| Rate of change in elongation at break | (%) | +18 | -94 | +44 | +6 | -84 | +89 |
| Compression set test | | | | | | | |
| (150°C, 70 hours) | (%) | 25 | 24 | 24 | 14 | 14 | 15 |
| (175°C, 70 hours) | (%) | 41 | 43 | 40 | 26 | 28 | 25 |

Example 16

A copolymerization reaction was performed in the same manner as in Example 1, except that the following charged monomer mixture was used, thereby obtaining acrylic rubber K. The Mooney viscosity $PML_{1+4}$ (100°C) of the obtained acrylic rubber K was 36.

Charged monomer mixture

| | |
|---|---|
| Ethyl acrylate [EA] | 97.25 parts by weight |
| Vinyl chloroacetate [VCA] | 2.5 parts by weight |
| Compound (e) of Reference Example 5 | 0.25 parts by weight |

The mole fraction compositions were as follows: compound (e): 0.096 mol%, EA+VCA: 99.904 mol%.

Further, the approximate weight fraction compositions were as follows: compound (e): 0.23 wt.%, EA+VCA: 99.77 wt.%.

Example 17

A copolymerization reaction was performed in the same manner as in Example 1, except that the following charged monomer mixture was used, thereby obtaining acrylic rubber L. The Mooney viscosity $PML_{1+4}$ (100°C) of the obtained acrylic rubber L was 38.

Charged monomer mixture

| | |
|---|---|
| Ethyl acrylate [EA] | 97.4 parts by weight |
| Vinyl chloroacetate [VCA] | 2.5 parts by weight |
| Compound (e) of Reference Example 5 | 0.1 parts by weight |

The mole fraction compositions were as follows: compound (e): 0.047 mol%, EA+VCA: 99.953 mol%.

Further, the approximate weight fraction compositions were as follows: compound (e): 0.11 wt.%, EA+VCA: 99.89 wt.%.

Example 18

In Example 14, the acrylic rubber K was used in place of the acrylic rubber H. Further, an oil dipping-air heating aging combined test was performed.

Oil dipping-air heating aging combined test:
After dipping in oil (IRM 903 oil) at 150°C for 168 hours according to JIS K6258, an air heating aging test was performed at 175°C for 300 hours according to JIS K6257, and the normal state physical properties were compared before and after oil dipping.

Example 19

In Example 14, the acrylic rubber L was used in place of the acrylic rubber H.

Comparative Example 12

In Comparative Example 8, 2.0 part by weight of 4,4'-bis(α,α-dimethylbenzyl)diphenylamine (Nocrac CD) was further added.

Following Table 5 shows the results obtained in the above Examples 18 to 19 and Comparative Example 12.

Table 5

| Measurement results | | | Ex. 18 | Ex. 19 | Comp. Ex. 12 |
|---|---|---|---|---|---|
| Mooney scorch test (125°C) | | | | | |
| | ML min | (pts) | 66 | 65 | 64 |
| | t5 | (min) | 6.3 | 6.2 | 6.4 |
| Crosslinking test (180°C) | | | | | |
| | tc (10) | (min) | 0.86 | 0.86 | 0.82 |
| | tc (90) | (min) | 4.83 | 4.74 | 4.94 |
| | ML | (N•m) | 0.31 | 0.32 | 0.27 |

(continued)

| | | | | | |
|---|---|---|---|---|---|
| Crosslinking test (180°C) | | | | | |
| | MH | (N•m) | 1.16 | 1.21 | 1.09 |
| Normal state physical properties | | | | | |
| | Hardness | (Duro A) | 67 | 69 | 65 |
| | 100% modulus | (MPa) | 6.2 | 6.2 | 5.6 |
| | Strength at break | (MPa) | 15.8 | 15.2 | 14.8 |
| | Elongation at break | (%) | 290 | 270 | 300 |
| Air heating aging test (175°C, 100 hours) | | | | | |
| | Hardness change | (Duro A) | +9 | +6 | +8 |
| | Rate of change in 100% modulus | (%) | -34 | -42 | -46 |
| | Rate of change in strength at break | (%) | -29 | -39 | -38 |
| | Rate of change in elongation at break | (%) | +8 | +22 | +38 |
| Air heating aging test (175°C, 200 hours) | | | | | |
| | Hardness change | (Duro A) | +7 | +8 | +7 |
| | Rate of change in 100% modulus | (%) | -55 | -66 | -63 |
| | Rate of change in strength at break | (%) | -52 | -74 | -61 |
| | Rate of change in elongation at break | (%) | +22 | +50 | +62 |
| Air heating aging test (175°C, 300 hours) | | | | | |
| | Hardness change | (Duro A) | +11 | +8 | +9 |
| | Rate of change in 100% modulus | (%) | -61 | -68 | -68 |
| | Rate of change in strength at break | (%) | -73 | -84 | -78 |
| | Rate of change in elongation at break | (%) | +31 | +54 | +88 |
| Air heating aging test (175°C, 400 hours) | | | | | |
| | Hardness change | (Duro A) | +13 | +14 | +11 |
| | Rate of change in 100% modulus | (%) | -61 | -60 | -71 |
| | Rate of change in strength at break | (%) | -81 | -80 | -83 |
| | Rate of change in elongation at break | (%) | +26 | +13 | +83 |
| Air heating aging test (175°C, 500 hours) | | | | | |
| | Hardness change | (Duro A) | +13 | +16 | +9 |
| | Rate of change in 100% modulus | (%) | -55 | -44 | -73 |
| | Rate of change in strength at break | (%) | -77 | -76 | -87 |
| | Rate of change in elongation at break | (%) | +2 | -30 | +72 |
| Oil dipping-air heating aging combined test | | | | | |
| | Hardness change¥ | (Duro A) | +12 | +11 | +17 |
| | Rate of change in 100% modulus | (%) | -55 | -63 | -52 |
| | Rate of change in strength at break | (%) | -71 | -82 | -78 |
| | Rate of change in elongation at break | (%) | +20 | +41 | -12 |
| Compression set test (150°C, 70 hours) | | (%) | 24 | 24 | 25 |

Example 20

A copolymerization reaction was performed in the same manner as in Example 11, except that the following charged monomer mixture was used, thereby obtaining acrylic rubber M. The Mooney viscosity $PML_{1+4}$ (100°C) of the obtained acrylic rubber M was 32.

Charged monomer mixture

| | |
|---|---|
| Ethyl acrylate [EA] | 98.2 parts by weight |

(continued)

| | |
|---|---|
| Mono n-butyl fumarate [MBF] | 1.6 parts by weight |
| Compound (e) of Reference Example 5 | 0.2 parts by weight |

The mole fraction compositions were as follows: compound (e): 0.085 mol%, EA+MBF: 99.915 mol%.
Further, the approximate weight fraction compositions were as follows: compound (e): 0.20 wt.%, EA+MBF: 99.80 wt.%.

Example 21

A copolymerization reaction was performed in the same manner as in Example 11, except that the following charged monomer mixture was used, thereby obtaining acrylic rubber N. The Mooney viscosity $PML_{1+4}$ (100°C) of the obtained acrylic rubber N was 32.

Charged monomer mixture

| | |
|---|---|
| Ethyl acrylate [EA] | 98.3 parts by weight |
| Mono n-butyl fumarate [MBF] | 1.6 parts by weight |
| Compound (e) of Reference Example 5 | 0.1 parts by weight |

The mole fraction compositions were as follows: compound (e): 0.047 mol%, EA+MBF: 99.953 mol%.
Further, the approximate weight fraction compositions were as follows: compound (e): 0.11 wt.%, EA+MBF: 99.89 wt.%.

Example 22

The acrylic rubbers G, M, N, and E respectively obtained in Examples 11, 20, and 21, and Reference Comparative Example 1 were subjected to a heating test at 150°C for 3 hours in the air, and the Mooney viscosity $PML_{1+4}$ (100°C) was compared before and after the test. The obtained results are shown in Table 6 below.

Table 6

| Acrylic rubber | E | G | M | N |
|---|---|---|---|---|
| Approximate copolymerization amount of the compound (e) (wt%)é | 0 | 0.48 | 0.20 | 0.11 |
| $PML_{1+4}$ (100°C) before heating test | 32 | 30 | 32 | 32 |
| $PML_{1+4}$ (100°C) after heating test | 9 | 30 | 31 | 33 |

Example 23

A copolymerization reaction was performed in the same manner as in Example 1, except that the following charged monomer mixture was used, thereby obtaining acrylic rubber P. The Mooney viscosity $PML_{1+4}$ (100°C) of the obtained acrylic rubber P was 36.

Charged monomer mixture

| | |
|---|---|
| Ethyl acrylate [EA] | 97.3 parts by weight |
| Vinyl chloroacetate [VCA] | 2.5 parts by weight |
| Compound (e) of Reference Example 5 | 0.2 parts by weight |

The approximate mole fraction compositions were as follows: compound (e): 0.080 mol%, EA+VCA: 99.920 mol%.

Further, the approximate weight fraction compositions were as follows: compound (e): 0.19 wt.%, EA+VCA: 99.81 wt.%.

Example 24

The same test as in Example 22 was performed, except that the acrylic rubbers H, P, L, and J respectively obtained in Examples 13, 23, and 17, and Reference Comparative Example 2 were used. The obtained results are shown in Table 7 below.

Table 7

| Acrylic rubber | J | H | P | L |
|---|---|---|---|---|
| Approximate copolymerization amount of the compound (e) (wt%) | 0 | 0.51 | 0.19 | 0.11 |
| $PML_{1+4}$ (100°C) before heating test | 43 | 38 | 36 | 38 |
| $PML_{1+4}$ (100°C) after heating test | 8 | 34 | 34 | 33 |

Example 25

In Example 5, the following component was used.

| | |
|---|---|
| Acrylic rubber G | 100 parts by weight |
| FEF carbon black (Seast GSO) | 60 parts by weight |
| Stearic acid (TST) | 1 part by weight |
| Polyoxyethylene stearyl ether phosphate (Phosphanol RL-210) | 0.5 parts by weight |
| Stearyl amine (Farmin 80S, produced by Kao Corporation) | 1 part by weight |
| Crosslinking accelerator (Vulcofac ACT55) | 1 part by weight |
| Hexamethylenediamine carbamate (Cheminox AC6F) | 0.6 parts by weight |

Further, an oil dipping-air heating aging combined test was performed.

Oil dipping-air heating aging combined test:

After dipping in oil (IRM 903 oil) at 150°C for 168 hours according to JIS K6258, an air heating aging test was performed at 190°C for 200 hours according to JIS K6257, and the normal state physical properties were compared before and after oil dipping.

Example 26

In Example 25, the acrylic rubber M was used in place of the acrylic rubber G.

Example 27

In Example 25, the acrylic rubber N was used in place of the acrylic rubber G.

Comparative Example 13

In Example 25, the acrylic rubber E was used in place of the acrylic rubber G, and 2.0 part by weight of 4,4'-Bis($\alpha,\alpha$-dimethylbenzyl)diphenylamine (Nocrac CD) was further added.

Comparative Example 14

In Comparative Example 13, the amount of 4,4'-bis($\alpha,\alpha$-dimethylbenzyl)diphenylamine (Nocrac CD) added was changed from 2.0 parts by weight to 1.0 part by weight.

Comparative Example 15

In Comparative Example 13, the 4,4'-bis($\alpha,\alpha$-dimethylbenzyl)diphenylamine (Nocrac CD) was not used.

Following Table 8 shows the results obtained in the above Examples 25 to 27 and Comparative Examples 13

to 15.

Table 8

| Measurement results | | Ex. 25 | Ex. 26 | Ex. 27 | Comp. Ex. 13 | Comp. Ex. 14 | Comp. Ex. 15 |
|---|---|---|---|---|---|---|---|
| Mooney scorch test (125°C) | | | | | | | |
| ML min | (pts) | 65 | 62 | 62 | 61 | 62 | 64 |
| t5 | (min) | 4.2 | 3.9 | 4.2 | 4.2 | 4.2 | 3.8 |
| Crosslinking test (180°C) | | | | | | | |
| tc (10) | (min) | 0.62 | 0.59 | 0.59 | 0.61 | 0.62 | 0.51 |
| tc (90) | (min) | 6.68 | 6.62 | 6.54 | 6.53 | 6.56 | 6.40 |
| ML | (N•m) | 0.31 | 0.30 | 0.31 | 0.27 | 0.28 | 0.27 |
| MH | (N•m) | 0.94 | 0.98 | 1.00 | 0.93 | 0.96 | 0.99 |
| Normal state physical properties | | | | | | | |
| Hardness | (Duro A) | 70 | 70 | 69 | 67 | 70 | 69 |
| 100% modulus | (MPa) | 5.8 | 5.6 | 5.6 | 4.8 | 4.9 | 4.9 |
| Strength at break | (MPa) | 16.1 | 15.9 | 16.0 | 15.1 | 15.0 | 16.0 |
| Elongation at break | (%) | 280 | 270 | 260 | 280 | 270 | 260 |
| Air heating aging test (175°C, 200 hours) | | | | | | | |
| Hardness change | (Duro A) | +13 | +10 | +8 | +12 | +6 | +8 |
| Rate of change in 100% modulus | (%) | +31 | +8 | -2 | -21 | -8 | -18 |
| Rate of change in strength at break | (%) | -7 | -9 | -18 | -28 | -20 | -32 |
| Rate of change in elongation at break | (%) | -24 | -10 | +0 | +18 | +10 | +7 |
| Air heating aging test (175°C, 400 hours) | | | | | | | |
| Hardness change | (Duro A) | +13 | +12 | +12 | +10 | +8 | +10 |
| Rate of change in 100% modulus | (%) | +17 | -11 | -23 | -29 | -27 | -29 |
| Rate of change in strength at break | (%) | -24 | -31 | -44 | -41 | -37 | -58 |
| Rate of change in elongation at break | (%) | -26 | -8 | -2 | +17 | +16 | +0 |
| Air heating aging test (175°C, 600 hours) | | | | | | | |
| Hardness change | (Duro A) | +12 | +12 | +15 | +12 | +11 | +17 |
| Rate of change in 100% modulus | (%) | -5 | -25 | -23 | -35 | -33 | -2 |
| Rate of change in strength at break | (%) | -45 | -57 | -63 | -59 | -58 | -63 |
| Rate of change in elongation at break | (%) | -22 | -7 | -14 | +25 | +15 | -36 |
| Air heating aging test (175°C, 800 hours) | | | | | | | |
| Hardness change | (Duro A) | +17 | +17 | +20 | +16 | +14 | +25 |
| Rate of change in 100% modulus | (%) | -9 | -9 | +2 | -38 | -22 | |
| Rate of change in strength at break | (%) | -58 | -62 | -61 | -69 | -65 | -58 |
| Rate of change in elongation at break | (%) | -35 | -37 | -51 | +12 | -7 | -68 |
| Air heating aging test (190°C, 100 hours) | | | | | | | |
| Hardness change | (Duro A) | +11 | +10 | +10 | +9 | +7 | +10 |

(continued)

| | | | | | | | |
|---|---|---|---|---|---|---|---|
| Air heating aging test (190°C, 100 hours) | | | | | | | |
| Rate of change in 100% modulus | (%) | +16 | -7 | -11 | -25 | -24 | -27 |
| Rate of change in strength at break | (%) | -14 | -21 | -29 | -33 | -29 | -49 |
| Rate of change in elongation at break | (%) | -19 | -8 | -4 | +15 | +12 | +5 |
| Air heating aging test (190°C, 200 hours) | | | | | | | |
| Hardness change | (Duro A) | +13 | +13 | +15 | +14 | +11 | +15 |
| Rate of change in 100% modulus | (%) | -5 | -25 | -25 | -35 | -29 | -4 |
| Rate of change in strength at break | (%) | -42 | -54 | -63 | -61 | -59 | -66 |
| Rate of change in elongation at break | (%) | -23 | -14 | -27 | +17 | +2 | -48 |
| Oil dipping-air heating aging combined test | | | | | | | |
| Hardness change | (Duro A) | +15 | +13 | +16 | +18 | +14 | +15 |
| Rate of change in 100% modulus | (%) | +10 | -13 | -20 | -13 | -10 | -14 |
| Rate of change in strength at break | (%) | -32 | -41 | -51 | -58 | -55 | -59 |
| Rate of change in elongation at break | (%) | -25 | -10 | -11 | -23 | -22 | -24 |
| Compression set test | | | | | | | |
| 175°C, 70 hours | (%) | 23 | 21 | 21 | 20 | 22 | 22 |
| 175°C, 500 hours | (%) | 41 | 38 | 38 | 36 | 37 | 38 |

The above results reveal the following.

(1) The air heating aging test data in Examples 5 to 8 show that the acrylic rubber crosslinked products copolymerized with a copolymerizable antioxidant are stabilized against thermal oxidative deterioration.
In contrast, in Comparative Example 1, in which no copolymerizable antioxidant is copolymerized, the rate of change in elongation at break rapidly decreases after 100 hours in the air heating aging test at 190°C (Figs. 1 to 4).
(2) In the oil dipping test, no significant difference is observed in the changes in mechanical properties between the Examples and the Comparative Examples. This suggests that thermal oxidative deterioration does not progress in the oil. The slight changes in physical properties are considered to be due to swelling of the crosslinked products by the oil component and various additives contained therein (Table 1).
(3) The reason for the fact that in the oil dipping-air heating aging combined test, the rate of change in elongation at break of Comparative Example 2 is significantly lower than that of each Example and is almost at the same level as Comparative Example 1 is presumed to be that 4,4'-bis($\alpha$,$\alpha$-dimethylbenzyl)diphenylamine as the antioxidant is extracted from the crosslinked product due to oil dipping.
On the other hand, in Examples 5 to 8, it is considered that the antioxidant component remained in the crosslinked products even after oil dipping, and that in the subsequent air heating aging test, the decrease in the rate of change in elongation at break was suppressed to a minimum due to its anti-heat aging properties (Figs. 5 and 6).
(4) In the air heating aging test, a comparison of the rate of change in elongation at break between Example 10 and Comparative Example 3 reveals that the acrylic rubber crosslinked product copolymerized with the copolymerizable antioxidant (e) is stabilized against thermal oxidative deterioration.
Furthermore, at the initial stage of this test, the rate of change in strength at break of Example 10 decreases more slowly than Comparative Examples 3 and 4, indicating that softening deterioration is suppressed. This is presumed to be due to the crosslinking effect of the copolymerizable antioxidant as the component (e) in the acrylic rubber. These results indicate that the copolymerizable antioxidant (e) has not only anti-aging properties but also a crosslinking effect (Figs. 7 and 8).
(5) In the oil dipping-air heating aging combined test, the decrease in the rate of change in strength at break of Example 10 is smaller than that of Comparative Examples 3 and 4, which is presumed to be due to the crosslinking

effect of the copolymerizable antioxidant as the component (e) in the acrylic rubber (Figs. 9 and 10).

(6) In the air heating aging-oil dipping-air heating aging combined test, the rate of change in elongation at break of Comparative Examples 3 and 4 is significantly lower than that of Example 10, the reason of which is presumed to be that 4,4'-bis(α,α-dimethylbenzyl)diphenylamine as the antioxidant is extracted from the crosslinked product due to oil dipping.

In addition, in this combined test, the smaller rate of change in strength at break of Example 10 than that of Comparative Examples 3 and 4 is presumed to be due to the crosslinking effect of the copolymerizable antioxidant as the component (e) (Figs. 11 and 12).

(7) These results are considered to be because the copolymerizable antioxidant component used in the present invention is chemically bonded to the rubber molecular chain, thereby suppressing the extraction of the antioxidant component due to oil dipping. This indicates the effectiveness of copolymerizing copolymerizable antioxidants into acrylic elastomer copolymers.

(8) When an acrylic elastomer copolymer in which the compound (e) is copolymerized is used, the elongation at break decreases slowly, indicating that this copolymer is stabilized against thermal oxidative deterioration (Example 12). On the other hand, when the compound (b-1) or the compound (e) is directly compounded into acrylic elastomer copolymers in which the compound (e) is not copolymerized (Comparative Examples 6 and 7), heat aging properties are merely almost equivalent to those when an antioxidant component is not compounded (Comparative Example 5). This indicates that the compound (b-1) and the compound (e) themselves do not have anti-heat aging properties. That is, although the compound (e) itself does not exhibit anti-heat aging properties, anti-heat aging properties are exhibited by copolymerization with a (meth)acrylate monomer, and as a result, the acrylic elastomer copolymer can be stabilized against thermal oxidative deterioration.

(9) When the crosslinking site monomer is an active chlorine-containing unsaturated monomer, the acrylic elastomer copolymer is stabilized against thermal oxidative deterioration by copolymerization of the compound (e), as shown in the results of the air heating aging test (Examples 14 and 15). In particular, due to the copolymerization of the compound (e), the softening deterioration of the acrylic elastomer copolymer crosslinked product can be significantly suppressed in the air heating aging test, as compared to 4,4'-bis(α,α-dimethylbenzyl)diphenylamine, which is a conventional antioxidant (Figs. 15 to 18).

(10) When the crosslinking site monomer is an active chlorine-containing unsaturated monomer, from the results of the heat aging test in Example 18 (amount of the compound (e) used during polymerization of the acrylic elastomer copolymer: 0.25 wt.%) and Comparative Example 12 (amount of the antioxidant 4,4'-bis(α,α-dimethylbenzyl)diphenylamine compounded: 2.0 parts by weight), the amount of antioxidant component used can be reduced by 80% or more. Furthermore, the anti-heat aging effect is also observed in Example 19 (amount of the compound (e) used during polymerization of the acrylic elastomer copolymer: 0.10 wt.%), in which the amount of antioxidant component is reduced by 95% compared to Comparative Example 12.

(11) Since an uncrosslinked acrylic elastomer copolymer in which the compound (e) is copolymerized is stabilized against thermal oxidative deterioration, the Mooney viscosity hardly changes during the heating test. On the other hand, in an uncrosslinked acrylic elastomer copolymer in which the compound (e) is not copolymerized, a significant decrease in the Mooney viscosity is observed (Tables 6 and 7). The significant decrease in the Mooney viscosity suggests that the main chain of the polymer is cleaved due to thermal oxidative deterioration, resulting in a decrease in the molecular weight.

(12) Even when the amount of the compound (e) used during polymerization of the acrylic elastomer copolymer is 0.10 to 0.50 wt.% (Examples 25 to 27), the crosslinked product is stabilized against thermal oxidative deterioration (Fig. 21).

## INDUSTRIAL APPLICABILITY

The acrylic elastomer copolymer of the present invention is effectively used as a material for acrylic elastomer molded members under various deterioration environments.

## Claims

1. An acrylic elastomer copolymer comprising a copolymerizable antioxidant represented by the general formula:

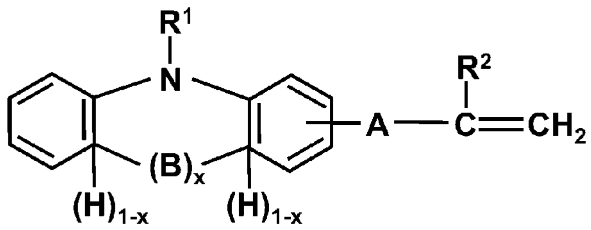

[I]

(wherein $R^1$ is a $C_{1-20}$ aliphatic hydrocarbon group, a $C_{7-20}$ aralkyl group, or a $C_{2-20}$ acyl group, $R^2$ is a hydrogen atom or a methyl group, A is a direct bond or a divalent organic group, B is a direct bond, an oxygen atom, a sulfur atom, a sulfoxide group, or a sulfone group, and x is 0 or 1), an alkyl (meth)acrylate monomer and/or an alkoxyalkyl (meth)acrylate monomer, and a crosslinking site monomer.

2. The acrylic elastomer copolymer according to claim 1, wherein in the copolymerizable antioxidant represented by the general formula [I], the divalent organic group is a group represented by the general formula $-(CH_2)_nO(C=O)-$ (wherein n is an integer of 1 to 5) or a group represented by the general formula $-NH(C=O)(CH_2)_nO(C=O)-$ (wherein n is an integer of 1 to 5).

3. The acrylic elastomer copolymer according to claim 1, wherein in the copolymerizable antioxidant is the general formula:

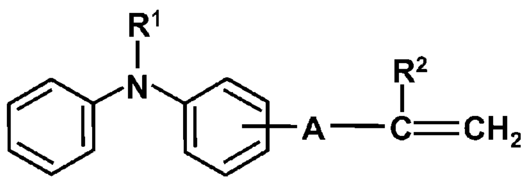

[II]

(wherein $R^1$ is a $C_{1-20}$ aliphatic hydrocarbon group, a $C_{7-20}$ aralkyl group, or a $C_{2-20}$ acyl group, $R^2$ is a hydrogen atom or a methyl group, A is a direct bond or a divalent organic group).

4. The acrylic elastomer copolymer according to claim 1, wherein in the copolymerizable antioxidant is the general formula:

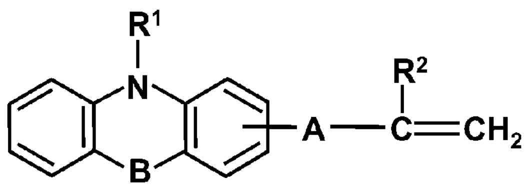

[III]

(wherein $R^1$ is a $C_{1-20}$ aliphatic hydrocarbon group, a $C_{7-20}$ aralkyl group, or a $C_{2-20}$ acyl group, $R^2$ is a hydrogen atom or a methyl group, A is a direct bond or a divalent organic group, B is a direct bond, an oxygen atom, a sulfur atom, a sulfoxide group, or a sulfone group).

5. The acrylic elastomer copolymer according to claim 4, wherein in the general formula [III], B is a sulfur atom, a sulfoxide group, or a sulfone group.

6. The acrylic elastomer copolymer according to claim 1, wherein the crosslinking site monomer is an $\alpha,\beta$-unsaturated carboxylic acid monomer.

7. The acrylic elastomer copolymer according to claim 1, wherein the crosslinking site monomer is an active chlorine-containing unsaturated monomer.

8. The acrylic elastomer copolymer according to claim 1, wherein the crosslinking site monomer is an epoxy group-containing unsaturated monomer.

**9.** A copolymerizable antioxidant for use in the acrylic elastomer copolymer according to claim 1, the copolymerizable antioxidant being represented by the general formula:

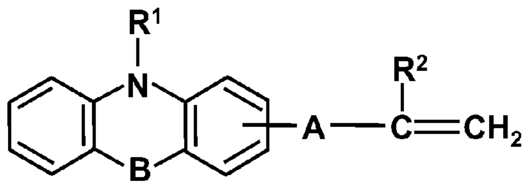

〔III〕

(wherein $R^1$ is a $C_{1-20}$ aliphatic hydrocarbon group, a $C_{7-20}$ aralkyl group, or a $C_{2-20}$ acyl group, $R^2$ is a hydrogen atom or a methyl group, A is a direct bond or a divalent organic group, B is a direct bond, an oxygen atom, a sulfur atom, a sulfoxide group, or a sulfone group).

**10.** A crosslinkable acrylic elastomer copolymer composition comprising the acrylic elastomer copolymer according to claim 6 compounded with a crosslinking agent.

**11.** A crosslinkable acrylic elastomer copolymer composition comprising the acrylic elastomer copolymer according to claim 7 compounded with a crosslinking agent.

**12.** A crosslinkable acrylic elastomer copolymer composition comprising the acrylic elastomer copolymer according to claim 8 compounded with a crosslinking agent.

**13.** The crosslinkable acrylic elastomer copolymer composition according to claim 10, wherein the crosslinking agent is a polyvalent amine compound.

**14.** The crosslinkable acrylic elastomer copolymer composition according to claim 11, wherein the crosslinking agent is 2,4,6-trimercapto-s-triazine.

**15.** The crosslinkable acrylic elastomer copolymer composition according to claim 11, wherein the crosslinking agent is a fatty acid alkali metal salt.

**16.** The crosslinkable acrylic elastomer copolymer composition according to claim 12, wherein the crosslinking agent is an organic acid ammonium salt.

**17.** The crosslinkable acrylic elastomer copolymer composition according to claim 12, wherein the crosslinking agent is a polyvalent amine compound.

[Fig. 1]

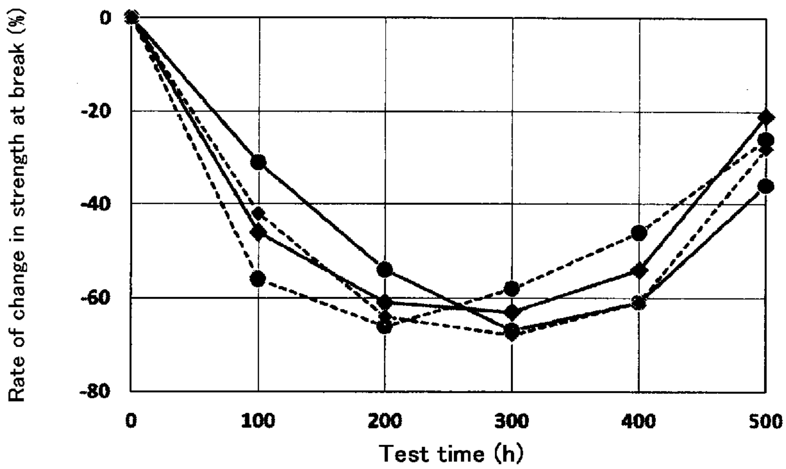

Rate of change in strength at break (%)
0
-20
-40
-60
-80
0
100
200
300
400
500
Test time (h)


[Fig. 2]

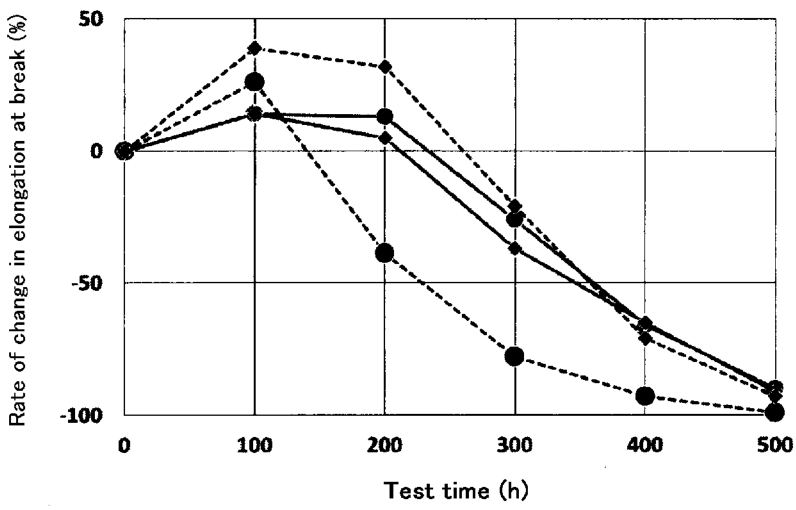

Rate of change in elongation at break (%)
50
0
-50
-100
0
100
200
300
400
500
Test time (h)

[Fig. 3]

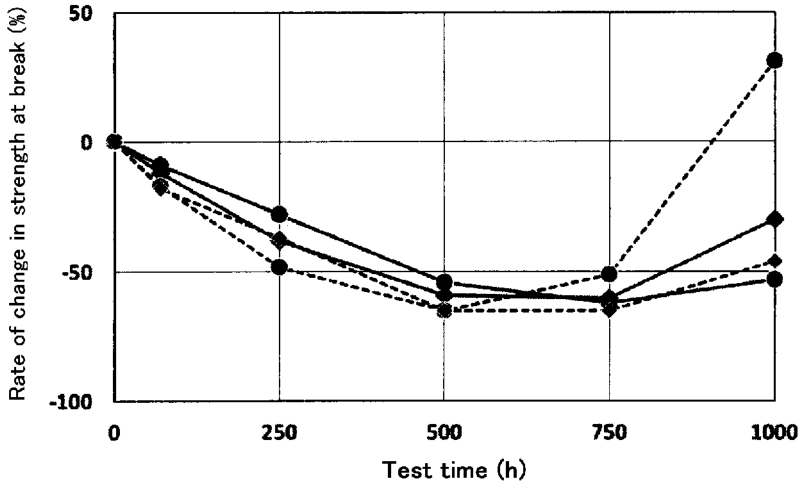
Rate of change in strength at break (%)
50
0
-50
-100
0
250
500
750
1000
Test time (h)

[Fig. 4]

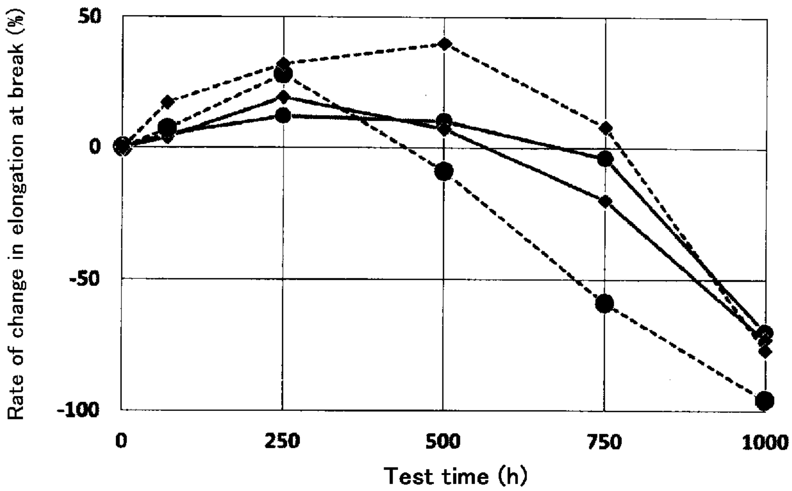
Rate of change in elongation at break (%)
50
0
-50
-100
0
250
500
750
1000
Test time (h)

[Fig. 5]

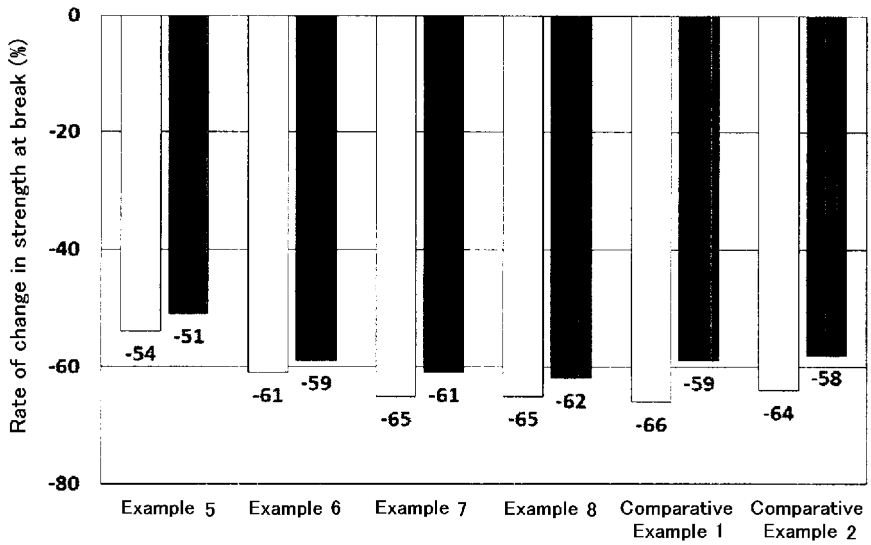
Rate of change in strength at break (%)
0
-20
-40
-60
-80
-54
-51
-61
-59
-65
-61
-65
-62
-66
-59
-64
-58
Example 5
Example 6
Example 7
Example 8
Comparative Example 1
Comparative Example 2

[Fig. 6]

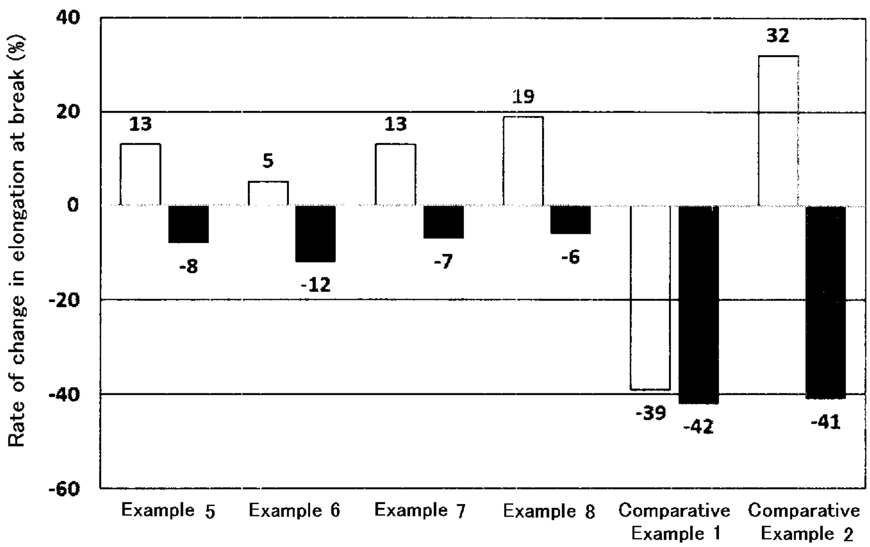
Rate of change in elongation at break (%)
40
20
0
-20
-40
-60
13
-8
5
-12
13
-7
19
-6
-39
-42
32
-41
Example 5
Example 6
Example 7
Example 8
Comparative Example 1
Comparative Example 2

[Fig. 7]

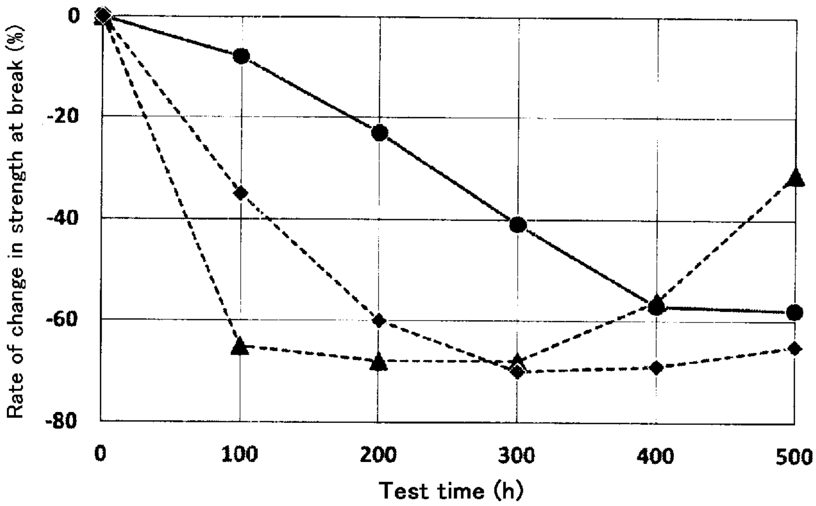
0
-20
-40
-60
-80
Rate of change in strength at break (%)
0
100
200
300
400
500
Test time (h)

[Fig. 8]

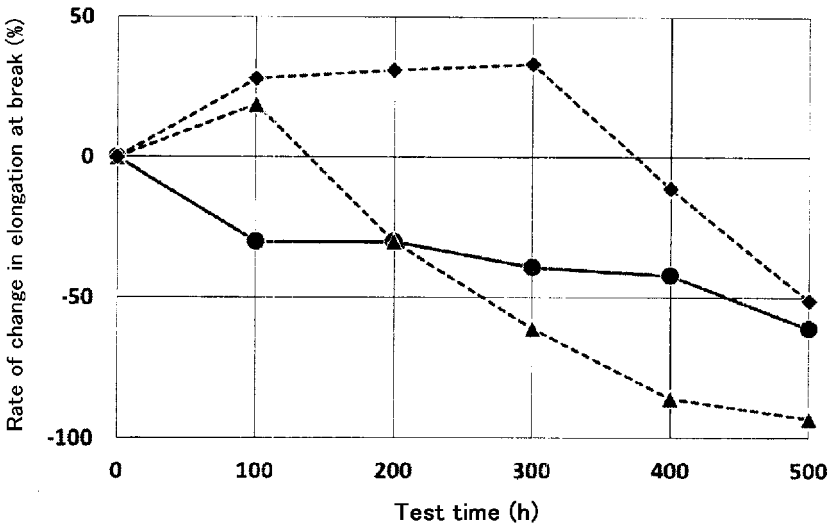
50
0
-50
-100
Rate of change in elongation at break (%)
0
100
200
300
400
500
Test time (h)

[Fig. 9]

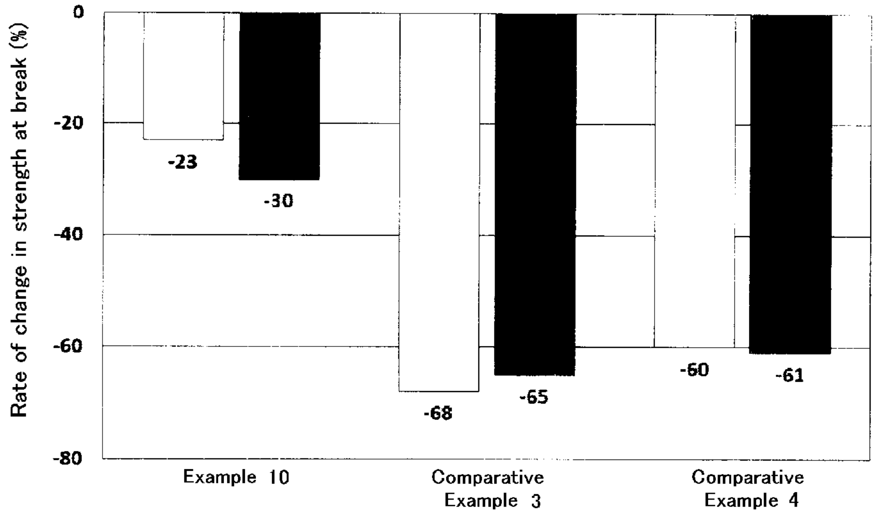
Rate of change in strength at break (%)
0
-20
-40
-60
-80
-23
-30
-68
-65
-60
-61
Example 10
Comparative Example 3
Comparative Example 4

[Fig. 10]

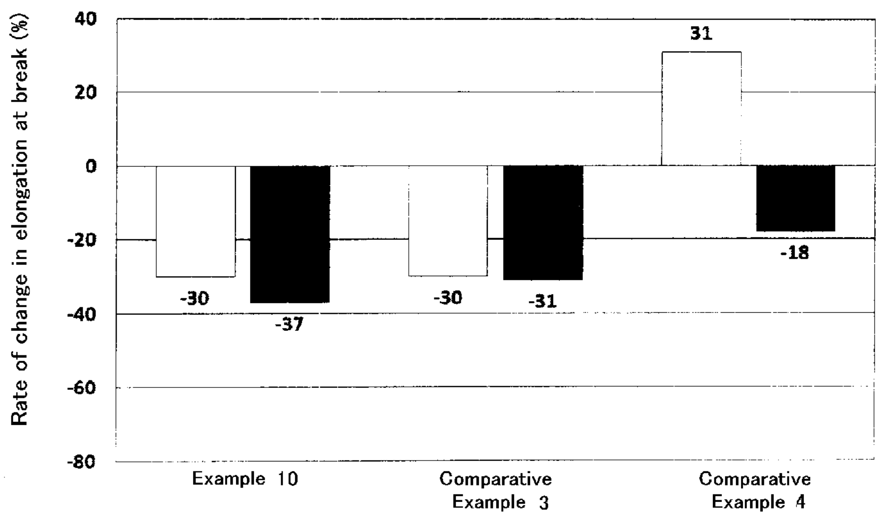
Rate of change in elongation at break (%)
40
20
0
-20
-40
-60
-80
-30
-37
-30
-31
31
-18
Example 10
Comparative Example 3
Comparative Example 4

[Fig. 11]

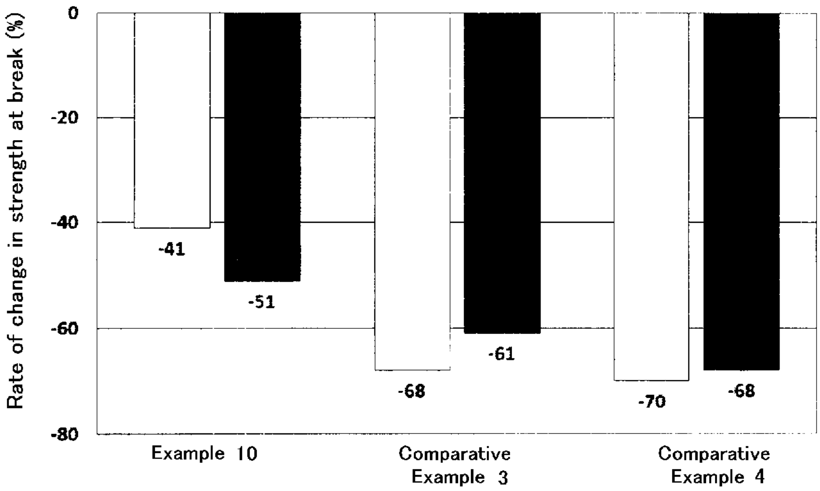
Rate of change in strength at break (%)
0
-20
-40
-60
-80
-41
-51
-68
-61
-70
-68
Example 10
Comparative Example 3
Comparative Example 4

[Fig. 12]

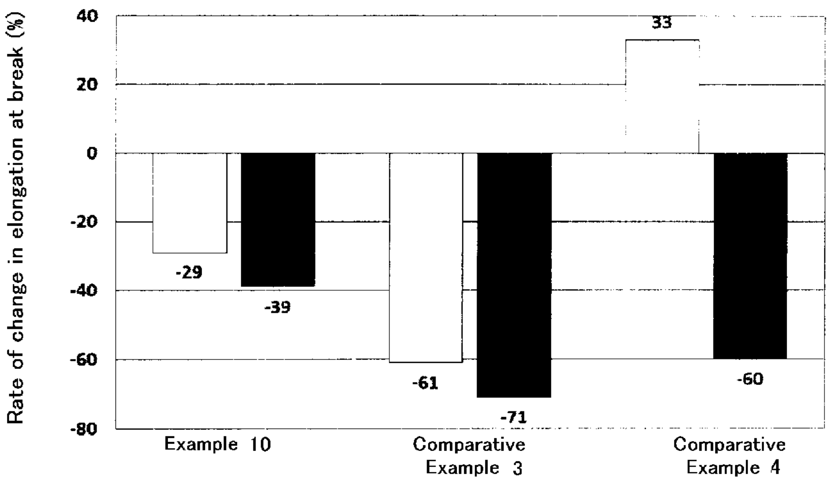
Rate of change in elongation at break (%)
40
20
0
-20
-40
-60
-80
33
-29
-39
-61
-71
-60
Example 10
Comparative Example 3
Comparative Example 4

[Fig. 13]

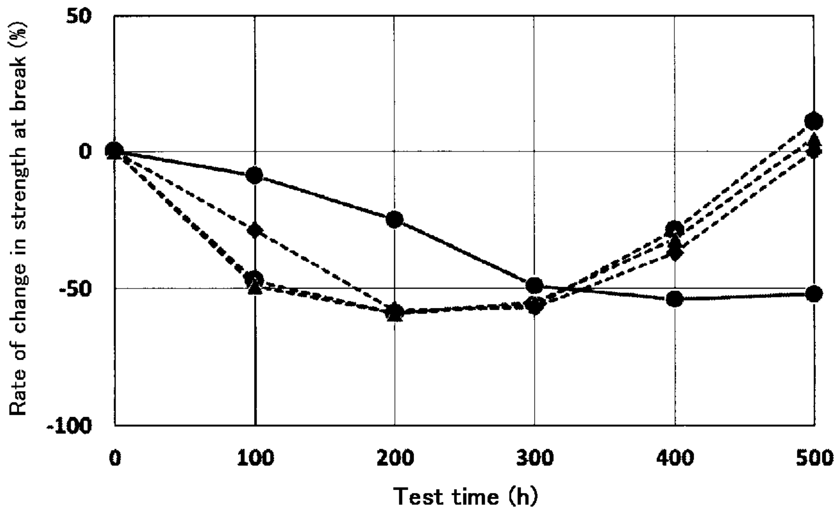
50
0
-50
-100
Rate of change in strength at break (%)
0
100
200
300
400
500
Test time (h)

[Fig. 14]

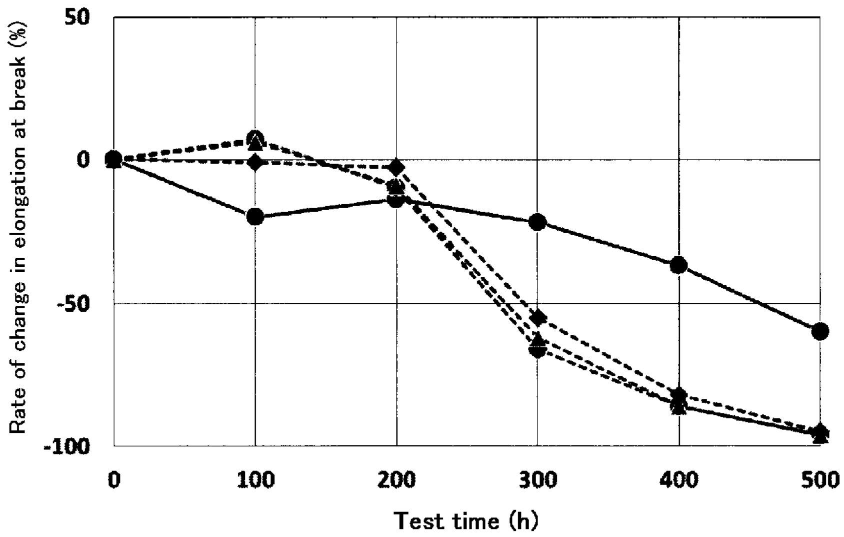
50
0
-50
-100
Rate of change in elongation at break (%)
0
100
200
300
400
500
Test time (h)

[Fig. 15]

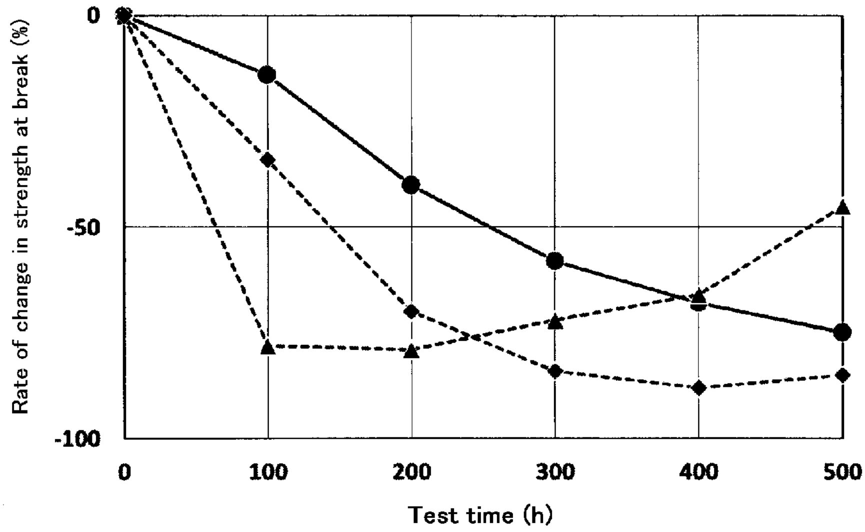
0
-50
-100
Rate of change in strength at break (%)
0
100
200
300
400
500
Test time (h)

[Fig. 16]

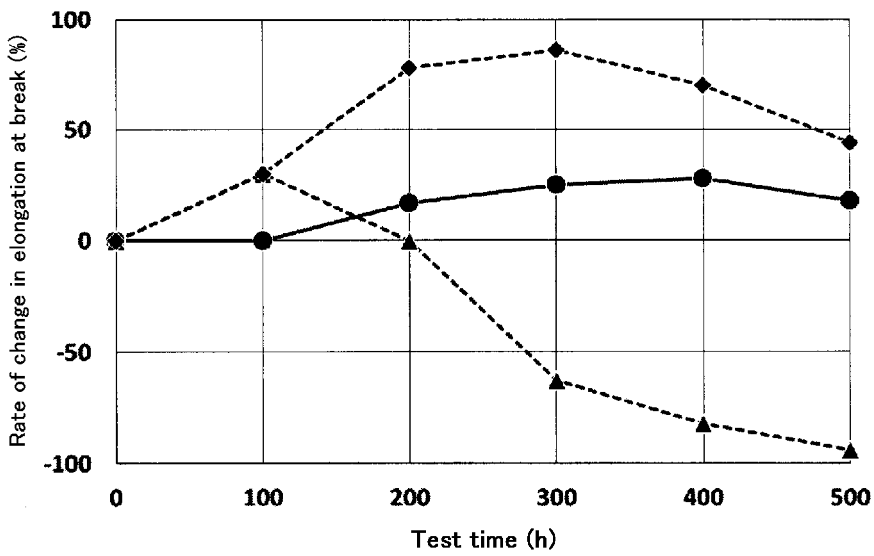
100
50
0
-50
-100
Rate of change in elongation at break (%)
0
100
200
300
400
500
Test time (h)

[Fig. 17]

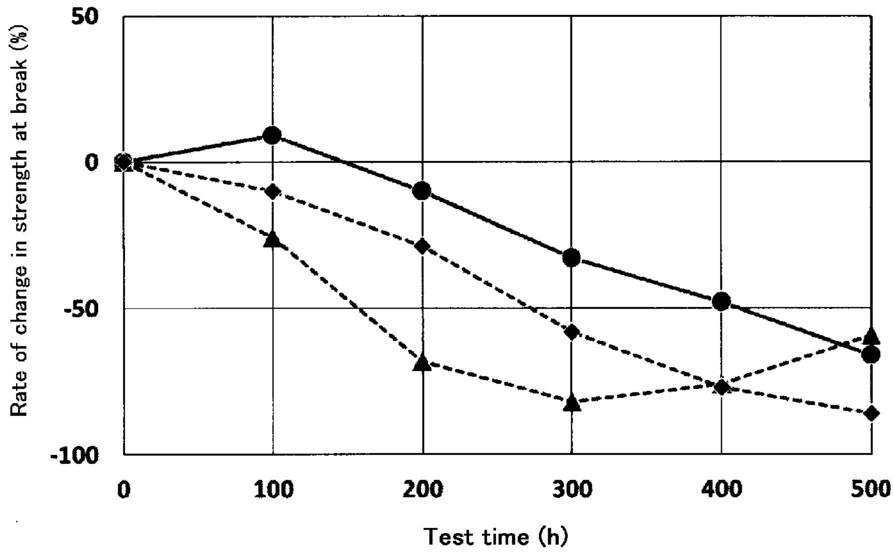
Rate of change in strength at break (%)
50
0
-50
-100
0
100
200
300
400
500
Test time (h)

[Fig. 18]

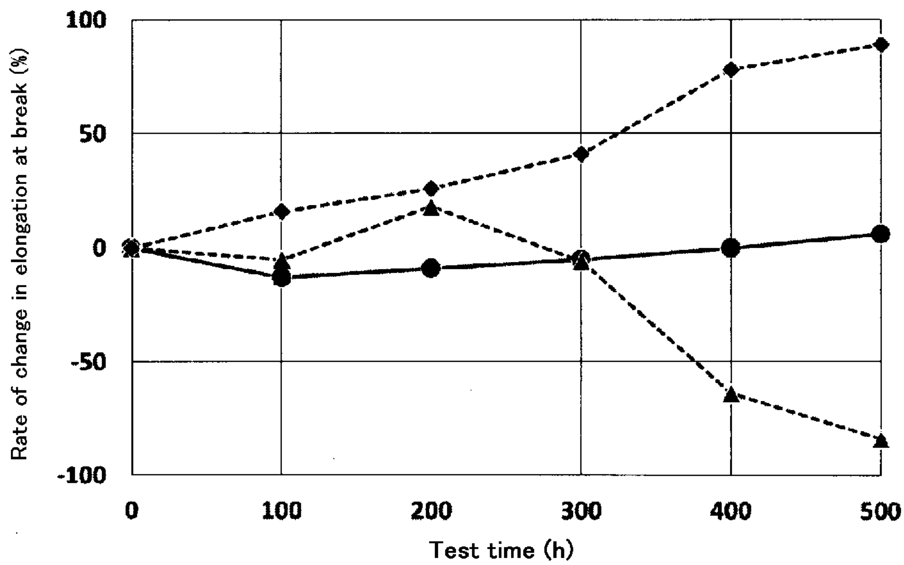
Rate of change in elongation at break (%)
100
50
0
-50
-100
0
100
200
300
400
500
Test time (h)

[Fig. 19]

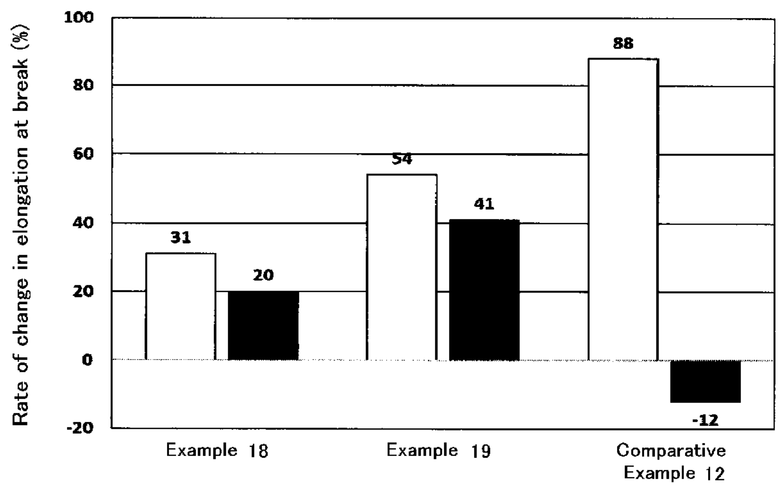
Rate of change in elongation at break (%)
100
80
60
40
20
0
-20
31
20
54
41
88
-12
Example 18
Example 19
Comparative Example 12

[Fig. 20]

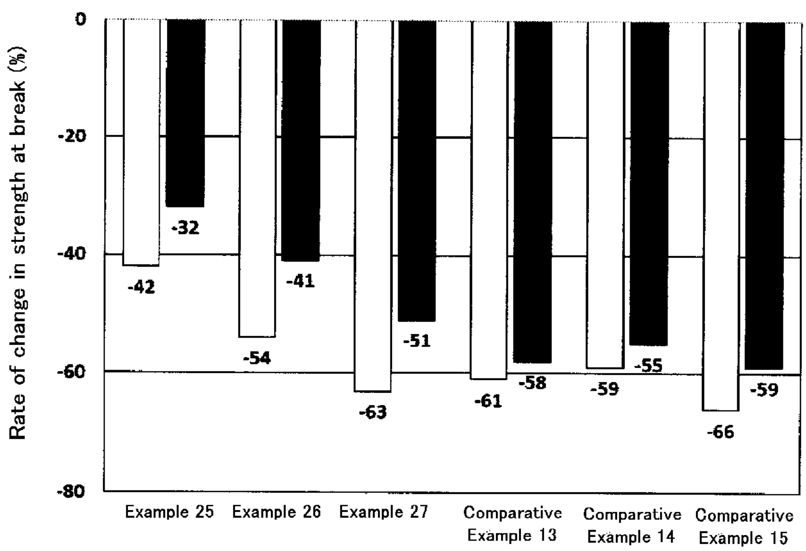
Rate of change in strength at break (%)
0
-20
-40
-60
-80
-42
-32
-54
-41
-63
-51
-61
-58
-59
-55
-66
-59
Example 25
Example 26
Example 27
Comparative Example 13
Comparative Example 14
Comparative Example 15

[Fig. 21]

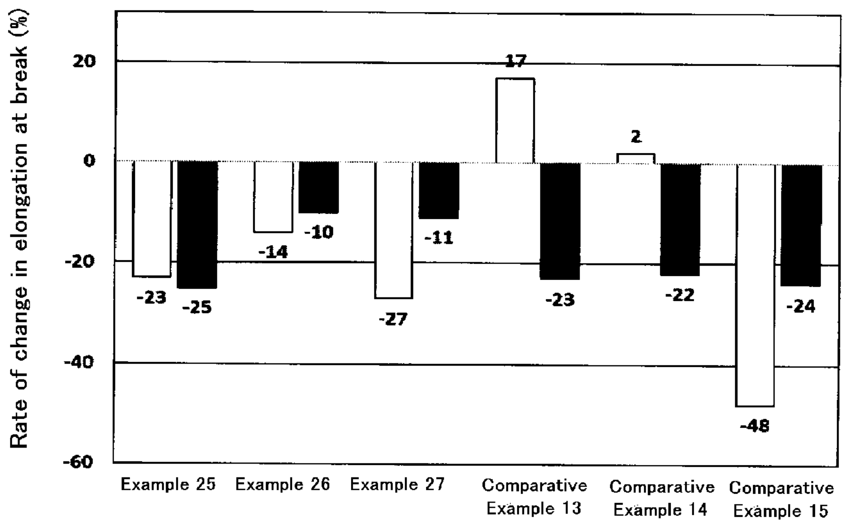
Rate of change in elongation at break (%)
20
0
-20
-40
-60
17
2
-23
-25
-14
-10
-27
-11
-23
-22
-48
-24
Example 25
Example 26
Example 27
Comparative Example 13
Comparative Example 14
Comparative Example 15

**INTERNATIONAL SEARCH REPORT**

International application No.
**PCT/JP2022/044383**

**A. CLASSIFICATION OF SUBJECT MATTER**

***C08F 220/10***(2006.01)i; ***C07D 279/36***(2006.01)i; ***C08F 212/32***(2006.01)i; ***C08K 5/17***(2006.01)i; ***C08L 31/06***(2006.01)i; ***C09K 15/32***(2006.01)i
FI: C08F220/10; C08L31/06; C08K5/17; C09K15/32 A; C07D279/36; C08F212/32

According to International Patent Classification (IPC) or to both national classification and IPC

**B. FIELDS SEARCHED**

Minimum documentation searched (classification system followed by classification symbols)

C08F220/10; C07D279/36; C08F212/32; C08K5/17; C08L31/06; C09K15/32

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2009-209268 A (NIPPON ZEON CO LTD) 17 September 2009 (2009-09-17) entire text | 1-17 |
| A | JP 2010-174217 A (DENKI KAGAKU KOGYO KK) 12 August 2010 (2010-08-12) entire text | 1-17 |
| A | JP 2020-111705 A (UNIMATEC CO LTD) 27 July 2020 (2020-07-27) entire text | 1-17 |
| A | JP 2011-32390 A (SEIKO KAGAKU KK) 17 February 2011 (2011-02-17) entire text | 1-17 |
| E, X | JP 2022-185177 A (UNIMATEC CO LTD) 14 December 2022 (2022-12-14) claims, examples | 1-17 |
| P, X | JP 2022-90304 A (UNIMATEC CO LTD) 17 June 2022 (2022-06-17) claims, examples | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 February 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/044383**

**C. DOCUMENTS CONSIDERED TO BE RELEVANT**

| Category* | Citation of document, with indication, where appropriate, of the relevant passages | Relevant to claim No. |
|---|---|---|
| P, X | JP 2021-187805 A (UNIMATEC CO LTD) 13 December 2021 (2021-12-13) claims, examples | 1-17 |

Form PCT/ISA/210 (second sheet) (January 2015)

**INTERNATIONAL SEARCH REPORT**
**Information on patent family members**

International application No.
**PCT/JP2022/044383**

| Patent document cited in search report | Publication date (day/month/year) | Patent family member(s) | Publication date (day/month/year) |
|---|---|---|---|
| JP 2009-209268 A | 17 September 2009 | (Family: none) | |
| JP 2010-174217 A | 12 August 2010 | (Family: none) | |
| JP 2020-111705 A | 27 July 2020 | (Family: none) | |
| JP 2011-32390 A | 17 February 2011 | (Family: none) | |
| JP 2022-185177 A | 14 December 2022 | (Family: none) | |
| JP 2022-90304 A | 17 June 2022 | (Family: none) | |
| JP 2021-187805 A | 13 December 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11021411 A
- WO 2007005458 A1
- JP 2010254579 A
- WO 2006001299 A1
- JP 2011032390 A
- WO 201158918 A1
- JP 2015227402 A
- WO 2011093443 A1
- JP 2009209268 A
- JP 4264106 A
- JP 5230132 A
- WO 2020158132 A1


### Non-patent literature cited in the description


- *Rubber Chem.Technol.,* 1973, vol. 46, 106
- *Rubber Chem.Technol.,* 1979, vol. 52, 883